# EUROPEAN PATENT APPLICATION

(11) **EP 1 156 123 A1**
(43) Date of publication of application: **21.11.2001**
(21) Application number: 00306004.3
(22) Date of filing: 14.07.2000
(51) Int. Cl.: C12Q 1/68

(54) **Method for detecting a variation of GH1 as indicator of growth hormone dysfunction**

(30) Priority: 12.05.2000 GB 0011459
(71) Applicant: University of Wales College of Medicine, Cardiff CF4 4XN (GB)
(72) Inventor: COOPER, David, Neil Department of Medical Genetics, Cardiff, CF14 4XN (GB); PROCTER, Anne Marie Departm. of Medical Genetics, Cardiff CF14 4XN (GB); GREGORY, John Department of Medical Genetics, Cardiff CF14 4XN (GB); MILLAR, David Stuart Dept. of Medical Genetics, Cardiff CF14 4XN (GB)
(74) Representative: Newell, William Joseph

(57) **Abstract**

A detection method for detecting a variation in *GH1* effective to act as an indicator of GH dysfunction in a patient, comprises the steps of comparing a test sample comprising a nucleotide sequence of the human *GH1* gene from the patient with a standard sequence known to be that of the human *GH1* gene. A difference between the test sample sequence and the standard sequence indicates the presence of a variation effective to act as an indicator of GH dysfunction (hereinafter "GH variant"). The test sample is obtained from a patient exhibiting the following cumulative criteria:
(i) height velocity below the 25^{th} centile for age;
(ii) bone age delay according to the Tanner-Whitehouse scale of at least two years when compared with chronological age; and
(iii) no other disorder known to cause inclusion in criteria (i) to (ii) above.

Also disclosed are mutations thereby detected, and their use in screening patients for growth hormone irregularities or for producing variant proteins suitable for treating such irregularities.

### Annex to the application documents - subsequently filed sequences listing

SEQUENCE LISTING

<110> University of Wales College of Medicine

<120> Sequences

<130> EPA 00306004.3

<140> EPA 00306004.3
<141> 2000-07-14

<160> 16

<170> PatentIn Ver. 2.1

<210> 1
<211> 1602
<212> DNA
<213> Homo sapiens

<400> 1
gatcttgtct cagaaaaacc ccagaaaaca acaacaaaaa aacgcgctat tgggcttatt 60
tctattttac gaaaatgagg aaccttggtc actgagaggt taagaatggg tgttgcctca 120
tgggtgctgt tggcatgtgg ggcagggaac tctgctttgc cagccaatgc ccctcccgcc 180
ccaggcaccc accggtcatt gagaccagcg cgattgccct gatgcatgtg cagacgctcc 240
ctccgttggg gcagagccac ccttagtaga gaaccactgg taacctgggg aggtttcctg 300
gctgctacac tgctgggtca ggactcaaac ccacgtcctt cttagcaggt cttttcccag 360
tcacagcagc ccagcagatc caggacacct gggctggaga aggaggcagg ggtctgagct 420
cttctttccc tgaggttctt ccccctcccg gcatcctagg gtggctgggg gtcgctgcct 480
tcttgcttcc ccaccccctg agccccttgt cccttcgccc gaccctggcc cccattcctt 540
attttgatgt tgccttcctg gaaagggagg tgtcgtcaga agtcagaatg tgaagccccg 600
ggcagccgag ggcagaggca ggggtaggct tgtgaggcct cctgttctgg gaagaaaagg 660
gcatcatgag cccaaaatgg ggctcagggc tgttgtcacc agtgagggaa cagacggctc 720
aaaatgtgac aaataaccct gcaggcggtg gggcccccgc tgcctccgcc cctcctgaaa 780
acagatccct gcccggcatg aatgggaatg aagcccaaag gccacatctg catccttgtc 840
cacgggatgt gtgtgtcttc cctccctgga gtgggagctg cttctcactt gtttttttaa 900
attttttatt tcaatatttt ctggggtaca ggtagttttt ttggttacct ggataagttc 960
tttagtggtg atttctgaga ttttagcgca cctgtcacct gaggatgggt gccaactgcc 1020
ccccaccaac ctccgtgccc cctacagtcc gctgagccct aggaccagct ccagagtagg 1080
ccccacacca cagggtccag gcaacacacc tgtgtgagca tgcacataca cacacacaca 1140
caccccccac aaacacgtgt gccccaagcc tttcccagtt atacccccca actttgggga 1200
gacactagcc ccaaagttaa tgaaggactc tgtcgttagg ggctcaggaa gagtatttcc 1260
taaaccatga gactcccaga ttttgcccca ctccccccgg gtcagtctct ctccagccac 1320
cctcaccagc atgcgggccc atgggcctca agctgacctc aggtgatgtt tatatttctg 1380
agctgtttat tccatgaact gaacatctga cagcttttca gagaaatgtt ttttcatttg 1440
gaacatctgg aaacaagaaa gaacatctgg ggctgcccgg aacgggctgt tcctcggatg 1500
aaaccttaac cctcctgccc ctgacttccg tggctcaggt cctggcctgc accccttgag 1560
agtggccccc accttatcag gtccgaggcc taggccaaga tc 1602

<210> 2
<211> 3700
<212> DNA
<213> Homo sapiens

<400> 2
ctgtttcttg gtttgtgtct ctgctgcaag tccaaggagc tggggcaata ccttgagtct 60
gggttcttcg tccccaggga cctgggggag ccccagcaat gctcagggaa aggggagagc 120
aaagtgtggg gttggttctc tctagtggtc agtgttggaa ctgcatccag ctgactcagg 180
ctgacccagg agtcctcagc agaagtggaa ttcaggactg aatcgtgctc acaaccccca 240
caatctattg gctgtgcttg gccccttttc ccaacacaca cattctgtct ggtgggtgga 300
ggttaaacat gcggggagga ggaaagggat aggatagaga atgggatgtg gtcggtaggg 360
ggtctcaagg actggctatc ctgacatcct tctccgcgtt caggttggcc accatggcct 420
gcggccagag ggcacccacg tgacccttaa agagaggaca agttgggtgg tatctctggc 480
tgacactctg tgcacaaccc tcacaacact ggtgacggtg ggaagggaaa gatgacaagc 540
cagggggcat gatcccagca tgtgtgggag gagcttctaa attatccatt agcacaagcc 600
cgtcagtggc cccatgcata aatgtacaca gaaacaggtg ggggcaacag tgggagagaa 660
ggggccaggg tataaaaagg gcccacaaga gaccagctca aggatcccaa ggcccaactc 720
cccgaaccac tcagggtcct gtggacagct cacctagcgg caatggctac aggtaagcgc 780
ccctaaaatc cctttgggca caatgtgtcc tgaggggaga ggcagcgacc tgtagatggg 840
acgggggcac taaccctcag gtttggggct tctgaatgtg agtatcgcca tgtaagccca 900
gtatttggcc aatctcagaa agctcctggt ccctggaggg atggagagag aaaaacaaac 960
agctcctgga gcagggagag tgctggcctc ttgctctccg gctccctctg ttgccctctg 1020
gtttctcccc aggctcccgg acgtccctgc tcctggcttt tggcctgctc tgcctgccct 1080
ggcttcaaga gggcagtgcc ttcccaacca ttcccttatc caggcttttt gacaacgcta 1140
tgctccgcgc ccatcgtctg caccagctgg cctttgacac ctaccaggag tttgtaagct 1200
cttggggaat gggtgcgcat caggggtggc aggaaggggt gactttcccc cgctgggaaa 1260
taagaggagg agactaagga gctcagggtt tttcccgaag cgaaaatgca ggcagatgag 1320
cacacgctga gtgaggttcc cagaaaagta acaatgggag ctggtctcca gcgtagacct 1380
tggtgggcgg tccttctcct aggaagaagc ctatatccca aaggaacaga agtattcatt 1440
cctgcagaac ccccagacct ccctctgttt ctcagagtct attccgacac cctccaacag 1500
ggaggaaaca caacagaaat ccgtgagtgg atgccttctc cccaggcggg gatgggggag 1560
acctgtagtc agagcccccg ggcagcacag ccaatgcccg tccttcccct gcagaaccta 1620
gagctgctcc gcatctccct gctgctcatc cagtcgtggc tggagcccgt gcagttcctc 1680
aggagtgtct tcgccaacag cctggtgtac ggcgcctctg acagcaacgt ctatgacctc 1740
ctaaaggacc tagaggaagg catccaaacg ctgatggggg tgagggtggc gccaggggtc 1800
cccaatcctg gagccccact gactttgaga gctgtgttag agaaacactg ctgccctctt 1860
tttagcagtc aggccctgac ccaagagaac tcaccttatt cttcatttcc cctcgtgaat 1920
cctccaggcc tttctctaca ccctgaaggg gagggaggaa aatgaatgaa tgagaaaggg 1980
agggaacagt acccaagcgc ttggcctctc cttctcttcc ttcactttgc agaggctgga 2040
agatggcagc ccccggactg ggcagatctt caagcagacc tacagcaagt tcgacacaaa 2100
ctcacacaac gatgacgcac tactcaagaa ctacgggctg ctctactgct tcaggaagga 2160
catggacaag gtcgagacat tcctgcgcat cgtgcagtgc cgctctgtgg agggcagctg 2220
tggcttctag ctgcccgggt ggcatccctg tgacccctcc ccagtgcctc tcctggccct 2280
ggaagttgcc actccagtgc ccaccagcct tgtcctaata aaattaagtt gcatcatttt 2340
gtctgactag gtgtccttct ataatattat ggggtggagg ggggtggtat ggagcaaggg 2400

gcaagttggg aagacaacct gtagggcctg cggggtctat tcgggaacca agctggagtg 2460
cagtggcaca atcttggctc actgcaatct ccgcctcctg ggttcaagcg attctcctgc 2520
ctcagcctcc cgagttgttg ggattccagg catgcatgac caggctcagc taatttttgt 2580
ttttttggta gagacggggt ttcaccatat tggccaggct ggtctccaac tcctaatctc 2640
aggtgatcta cccaccttgg cctcccaaat tgctgggatt acaggcgtga accactgctc 2700
ccttccctgt ccttctgatt ttaaaataac tataccagca ggaggacgtc cagacacagc 2760
ataggctacc tgccatgccc aaccggtggg acatttgagt tgcttgcttg gcactgtcct 2820
ctcatgcgtt gggtccactc agtagatgcc tgttgaattc ctgggcctag ggctgtgcca 2880
gctgcctcgt cccgtcacct tctggcttct tctctccctc catatcttag ctgttttcct 2940
catgagaatg ttccaaattc gaaatttcta tttaaccatt atatatttac ttgtttgcta 3000
ttatctctgc ccccagtaga ttgttagctc cagaagagaa aggatcatgt cttttgctta 3060
tctagatatg cccatctgcc tggtacaatc tctggcacat gttacaggca acaactactt 3120
gtggaattgg tgaatgcatg aatagaagaa tgagtgaatg aatgaataga caaaaggcag 3180
aaatccagcc tcaaagaact tacagtctgg taagaggaat aaaatgtctg caaatagcca 3240
caggacaggt caaaggaagg aggggctatt tccagctgag ggcaccccat caggaaagca 3300
ccccagactt cctacaacta ctagacacat ctcgatgctt ttcacttctc tatcaatgga 3360
tcgtctccct ggagaataat ccccaaagtg aaattactta gcacgtccag ttaggtagat 3420
ccttgtgtac ttcttggttg ttcagagatc atcaaccagt gcaaacaatc cccccatcaa 3480
tacacagcag tgcctgcccc tctccccccg aggtcttccg aggcccttcc tccgtgcctg 3540
aaccccctgg acatatcata tggcaaactg aagtgtccaa cgagatatag gaagtgaaac 3600
acgatgtaca ctgaaacgtg caatacaaat atgcagcatg aagtgcctcg gttcactaac 3660
ccgagctacg ctgggtgctt cttttctacc actttcctta 3700

<210> 3
<211> 654
<212> DNA
<213> Homo sapiens

<400> 3
atggctacag gctcccggac gtccctgctc ctggcttttg gcctgctctg cctgccctgg 60
cttcaagagg gcagtgcctt cccaaccatt cccttatcca ggctttttga caacgctagt 120
ctccgcgccc atcgtctgca ccagctggcc tttgacacct accaggagtt tgaagaagcc 180
tatatcccaa aggaacagaa gtattcattc ctgcagaacc cccagacctc cctctgtttc 240
tcagagtcta ttccgacacc ctccaacagg gaggaaacac aacagaaatc caacctagag 300
ctgctccgca tctccctgct gctcatccag tcgtggctgg agcccgtgca gttcctcagg 360
agtgtcttcg ccaacagcct ggtgtacggc gcctctgaca gcaacgtcta tgacctccta 420
aaggacctag aggaaggcat ccaaacgctg atggggaggc tggaagatgg cagcccccgg 480
actgggcaga tcttcaagca gacctacagc aagttcgaca caaactcaca caacgatgac 540
gcactactca agaactacgg gctgctctac tgcttcagga aggacatgga caaggtcgag 600
acattcctgc gcatcgtgca gtgccgctct gtggagggca gctgtggctt ctag 654

<210> 4
<211> 217
<212> PRT
<213> Homo sapiens

<400> 4

<210> 5
<211> 18
<212> DNA
<213> Homo sapiens

<400> 5
ctccgcgttc aggttggc                                                    18

<210> 6
<211> 18
<212> DNA
<213> Homo sapiens

<400> 6
aggtgagctg tccacagg                                                    18

<210> 7
<211> 18
<212> DNA
<213> Homo sapiens

<400> 7
cttccaggga ccaggagc                                                    18

<210> 8
<211> 22
<212> DNA
<213> Homo sapiens

<400> 8
catgtaagcc aagtatttgg cc                                               22

<210> 9
<211> 21
<212> DNA
<213> Homo sapiens

<400> 9
ggagaaggca tccactcacg g                                                21

<210> 10
<211> 21
<212> DNA
<213> Homo sapiens

<400> 10
tcagagtcta ttccgacacc c                                                21

<210> 11
<211> 26
<212> DNA
<213> Homo sapiens

<400> 11
cgtagttctt gagtagtgcg tcatcg                                          26


<210> 12
<211> 25
<212> DNA
<213> Homo sapiens

<400> 12
ttcaagcaga cctacagcaa gttcg                                           25


<210> 13
<211> 18
<212> DNA
<213> Homo sapiens

<400> 13
gtgccccaag cctttccc                                                   18


<210> 14
<211> 21
<212> DNA
<213> Homo sapiens

<400> 14
tgtcagatgt tcagttcatg g                                               21


<210> 15
<211> 18
<212> DNA
<213> Homo sapiens

<400> 15
cctcaagctg acctcagg                                                   18


<210> 16

<211> 19
<212> DNA
<213> Homo sapiens

<400> 16
gatcttggcc taggcctcg 19

## Description

The present invention relates to a method for detecting naturally-occurring growth hormone mutations; to mutations thereby detected and their use in screening patients for growth hormone irregularities or for producing variant proteins suitable for treating such irregularities.

That human stature was influenced by inherited factors was understood more than a century ago. Although familial short stature, with its normally recessive mode of inheritance, was recognised as early as 1912, it was a further quarter century before such families came to be properly documented in the scientific literature. The recognition that recessively inherited short stature was commonly associated with isolated growth hormone (GH) deficiency only came in 1966.

Short stature associated with GH deficiency has been estimated to occur with an incidence of between 1/4000 and 1/10000 live births. Most of these cases are both sporadic and idiopathic, but between 5 and 30% have an affected first-degree relative consistent with a genetic aetiology for the condition. Confirmation of the genetic aetiology of GH deficiency came from the molecular genetic analysis of familial short stature and the early demonstration of mutational lesions in the pituitary-expressed growth hormone (*GH1*) genes of affected individuals. Familial short stature may also be caused by mutation in a number of other genes (*eg POU1F1, PROP1* and *GHRHR*) and it is important to distinguish these different forms of the condition.

Growth hormone (GH) is a multifunctional hormone that promotes post-natal growth of skeletal and soft tissues through a variety of effects. Controversy remains as to the relative contribution of direct and indirect actions of GH. On one hand, the direct effects of GH have been demonstrated in a variety of tissues and organs, and GH receptors have been documented in a number of cell types. On the other hand, a substantial amount of data indicates that a major portion of the effects of GH are mediated through the actions of GH-dependent insulin-like growth factor I (IGF-I). IGF-1 is produced in many tissues, primarily the liver, and acts through its own receptor to enhance the proliferation and maturation of many tissues, including bone, cartilage, and skeletal muscle. In addition to promoting growth of tissues, GH has also been shown to exert a variety of other biological effects, including lactogenic, diabetogenic, lipolytic and protein anabolic effects, as well as sodium and water retention.

Adequate amounts of GH are needed throughout childhood to maintain normal growth. Newborns with GH deficiency are usually of normal length and weight. Some may have a micropenis or fasting hypoglycemia in conjunction with low linear postnatal growth, which becomes progressively retarded with age. In those with isolated growth hormone deficiency (IGHD), skeletal maturation is usually delayed in association with their height retardation. Truncal obesity, facial appearance younger than expected for their chronological age and delayed secondary dentition are often present. Skin changes similar to those seen in premature ageing may be seen in affected adults.

Familial IGHD comprises several different disorders with characteristic modes of inheritance. Those forms of IGHD known to be associated with defects at the *GH1* gene locus are shown in Table 1 together with the different types of underlying lesion so far detected.

**Table 1**

| **Classification of inherited disorders involving the *GH1* gene** | | | | |
|---|---|---|---|---|
| **Disorder** | **Mode of inheritance** | **Types of gene lesion responsible** | **GH protein** | **Deficiency state** |
| IGHD IA | Autosomal recessive | Gross deletions, micro-deletions, nonsense mutations | Absent | Severe short stature. Anti-GH antibodies often produced upon GH treatment, resulting in poor response thereto. |
| IGHD IB | Autosomal recessive | Splice site mutations | Deficient | Short stature. Patients usually respond well to exogenous GH |
| IGHD II | Autosomal dominant | Splice site and intronic mutations, missense mutations | Deficient | Short stature. Patients usually respond well to exogenous GH |

The characterisation of these lesions has helped to provide explanations for the differences in clinical severity, mode of inheritance and propensity to antibody formation in response to exogenously administered GH, between these forms of IGHD. Most cases are sporadic and are assumed to arise from cerebral insults or defects that include cerebral oedema, chromosomal anomalies, histiocytosis, infections, radiation, septo-optic dysplasia, trauma, or tumours affecting the hypothalamus or pituitary. Magnetic resonance imaging examinations detect hypothalamic or pituitary anomalies in about 12% of patients who have IGHD.

Although short stature, delayed 'height velocity' or growth velocity, and delayed skeletal maturation are all seen with GH deficiency, none of these is specific for this disorder; other systemic diseases may result in such symptoms. Throughout this specification, 'height velocity' and growth velocity are both to be construed as meaning the rate of change of the subject's or patient's height, such as is measured in centimetres *per* year.

Stimulation tests to demonstrate GH deficiency use L-Dopa, insulin-induced hypoglycaemia, arginine, insulin-arginine, clonidine, glucagon or propranolol. Inadequate GH peak responses (usually <7-10 ng/mL) differ from test to test. Testing for concomitant deficiencies of LH, FSH, TSH and ACTH should be performed to determine the extent of pituitary dysfunction and to plan optimal treatment.

Recombinant-derived GH is available worldwide and is administered by subcutaneous injection. To obtain an optimal outcome, children with IGHD are usually started on replacement therapy as soon as their diagnosis is established. The initial dosage of recombinant GH is based on body weight or surface area, but the exact amount used and the frequency of administration may vary between different protocols. The dosage increases with increasing body weight to a maximum during puberty. Thereafter, GH treatment should be temporarily discontinued while the individual's GH secretory capacity is re-evaluated. Those with confirmed GH deficiency receive a lower dose of exogenous GH during adult life.

Conditions that are treated with GH include (i) those in which it has proven efficacy and (ii) a variety of others in which its use has been reported but not accepted as standard practice. Disorders in which GH treatment has proven efficacy include GH deficiency, either isolated or in association with combined pituitary hormone deficiency (CPHD) and Turner syndrome. The clinical responses of individuals with the first two disorders to GH replacement therapy varies depending on (i) the severity of the GH deficiency and its adverse effects on growth, the age at which treatment is begun, weight at birth, current weight and dose of GH; and (ii) recognition and response to treatment of associated deficiencies such as thyroid hormone deficiency, and (iii) whether treatment is complicated by the development of anti-GH antibodies. The outcome of treatment for individuals with Turner syndrome subjects varies with the severity of their short stature, their chromosomal complement, and the age at which treatment was begun.

Additional disorders in which the use of GH has been reported include treatment of certain skeletal dysplasias such as achondroplasia, Prader-Willi syndrome, growth suppression secondary to exogenous steroids or in association with chronic inflammatory diseases such as rheumatoid arthritis, in chronic renal failure, extreme idiopathic short stature, Russell-Silver syndrome, and intrauterine growth retardation.

The characterisation of familial IGHD at the molecular genetic level is important for several reasons. The identity of the locus involved will indicate not only the likely severity of growth retardation but, more importantly, the appropriateness or otherwise of the various therapeutic regimens now available. Further, detection of the underlying gene lesions serves to confirm the genetic aetiology of the condition. It may also have prognostic value in predicting (i) the severity of growth retardation and (ii) the likelihood of anti-GH antibody formation subsequent to GH treatment. In some instances, knowledge of the pathological lesion(s) can also help to explain an unusual mode of inheritance of the disorder and is therefore essential for the counselling of affected families. Finally, the characterisation of the mutational lesions responsible for cases of IGHD manifesting a dysfunctional (as opposed to a non-functional) GH molecule could yield new insights into GH structure and function.

At the cellular level, a single GH molecule binds two GH receptor molecules (GHR) causing them to dimerise. Dimerisation of the two GH-bound GHR molecules is believed to be necessary for signal transduction, which is associated with the tyrosine kinase JAK-2. It has been suggested that the diverse effects of GH may be mediated by a single type of GHR molecule that can possess different cytoplasmic domains or phosphorylation sites in different tissues. When activated by JAK-2, these differing cytoplasmic domains can lead to distinct phosphorylation pathways, one for growth effects and others for various metabolic effects.

GH is a 22 kDa protein secreted by the somatotroph cells of the anterior pituitary. X-ray crystallographic studies have shown GH to comprise a core of two pairs of parallel alpha helices arranged in an up-up-down-down fashion. This structure is stabilised by two intra-molecular disulphide linkages (Cys53-Cys165 and Cys182-Cys 189). Two growth hormone receptor (GHR) molecules bind to two structurally distinct sites on the GH molecule, a process which proceeds sequentially by GHR binding first at site 1 and then at site 2. The binding of GHR to GH potentiates dimerisation of the GHR molecules.

Scanning mutagenesis studies of the GH molecule have yielded a picture of the binding interactions between GH and its receptor whilst site-directed mutagenesis has been used to probe the function of specific residues. Thus, substitution of Gly120 (in the third alpha helix of human GH) by Arg results in the loss of GHR binding to site 2 thereby blocking GHR dimerisation. Similarly, residue Phe44 of the human GH protein is important for binding the prolactin receptor. Finally, residues Asp 115, Gly119, Alal22 and Leu123 have been shown to be critical for the growth enhancing potential of the murine GH molecule.

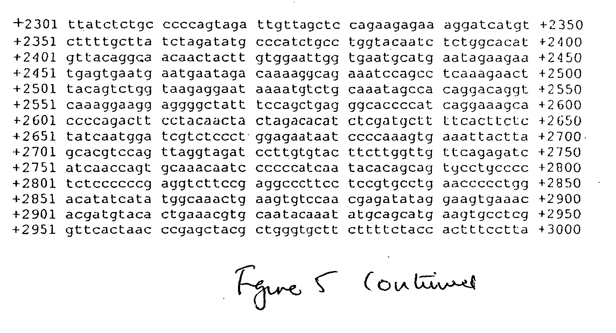

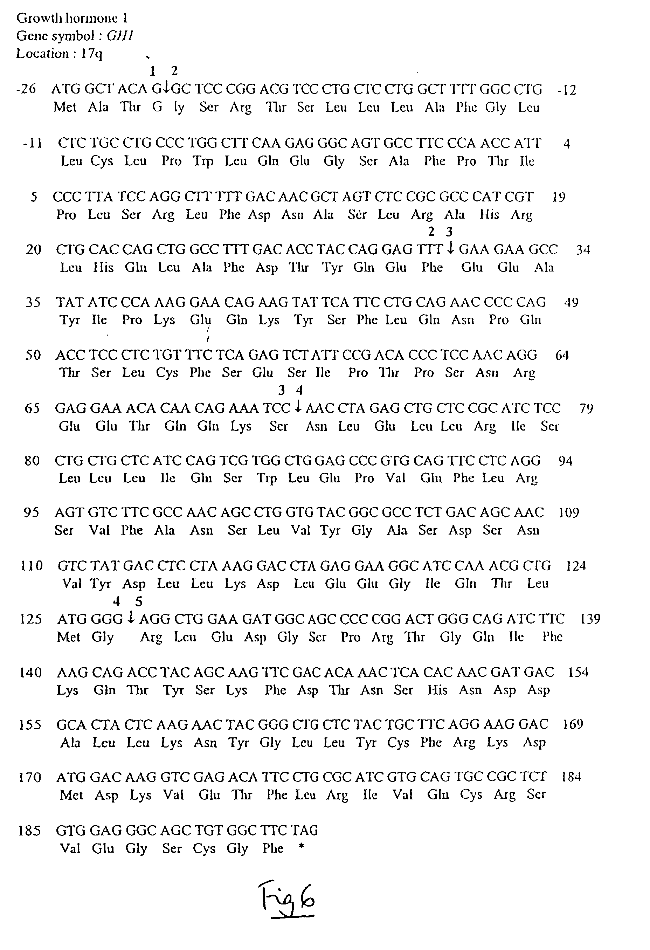

Interaction of the dimerised GHR with the intracellular tyrosine protein kinase JAK2 leads to tyrosine phosphorylation of downstream signal transduction molecules, stimulation of mitogen-activated protein (MAP) kinases and induction of signal transducers and activators of transcription (STAT proteins). In this way, GH is able to influence the expression of multiple genes through a number of different signalling pathways.
Several different GH isoforms are generated from expression of the *GH1* gene (*GH1* reference sequence is shown in Figure 5). In 9% of *GH1* transcripts, exon 2 is spliced to an alternative acceptor splice site 45bp into exon 3, thereby deleting amino acid residues 32 to 46 and generating a 20 kDa isoform instead of the normal 22 kDa protein. This 20 kDa isoform appears to be capable of stimulating growth and differentiation. The factors involved in determining alternative acceptor splice site selection are not yet characterised but are clearly of a complex nature. A 17.5 kDa isoform, resulting from the absence of codons 32 to 71 encoded by exon 3, has also been detected in trace amounts in pituitary tumour tissue. Splicing products lacking either exons 3 and 4 or exons 2, 3 and 4 have been reported in pituitary tissue but these appear to encode inactive protein products. A 24 kDa glycosylated variant of GH has also been described. The amino acid sequence of the major 22 kDa isoform is presented in Figure 6, which shows the nucleotide sequence of the *GH1* gene coding region and amino acid sequence of the protein including the 26 amino acid leader peptide. Lateral numbers refer to amino acid residue numbering. Numbers in bold flanking vertical arrows specify the exon boundaries. The termination codon is marked with an asterisk.

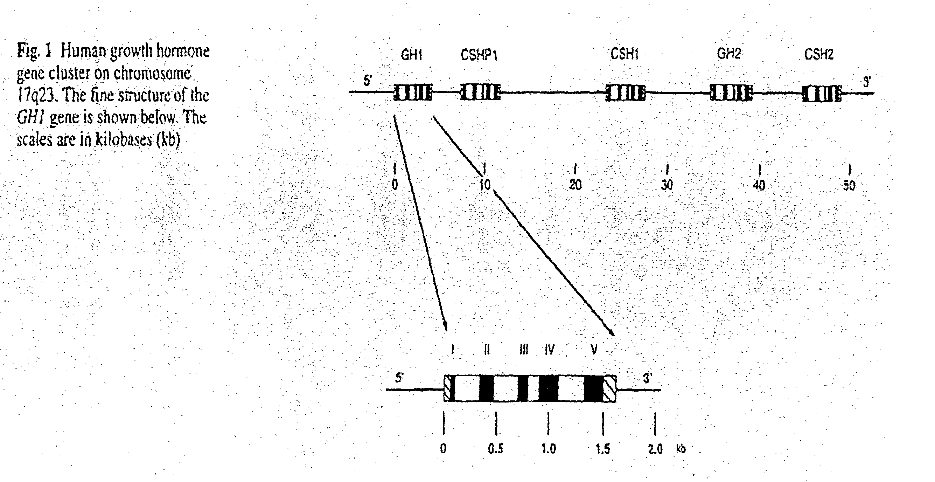

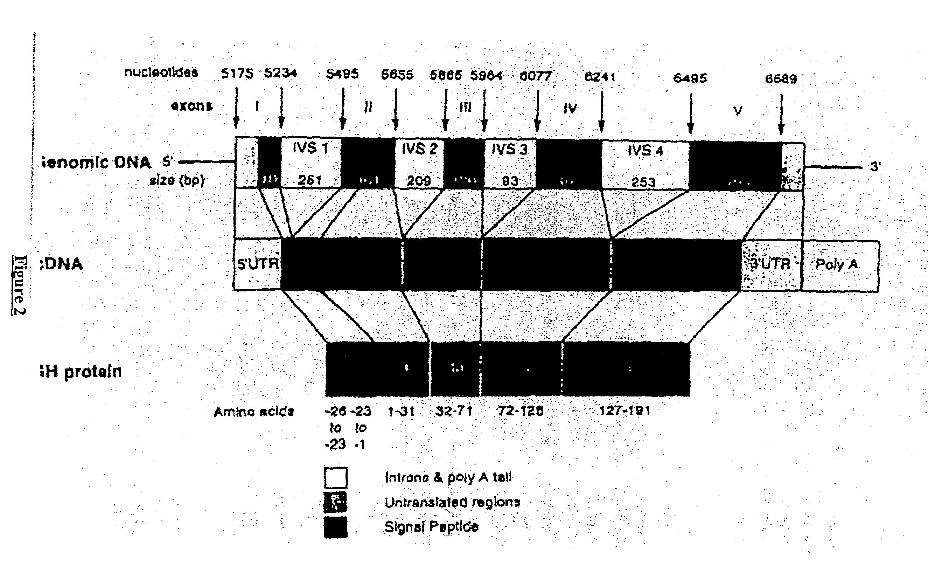

The gene encoding pituitary growth hormone (*GH1*) is located on chromosome 17q23 within a cluster of five related genes (Figure 1). This 66.5 kb cluster has now been sequenced in its entirety [Chen *et al. Genomics* 4: 479-497 (1989) and see Figure 5]. The other loci present in the growth hormone gene cluster are two chorionic somatomammotropin genes (*CSH1* and *CSH2*), a chorionic somatomammotropin pseudogene (*CSHP1*) and a growth hormone gene (*GH2*). These genes are separated by intergenic regions of 6 to 13 kb in length, lie in the same transcriptional orientation, are placentally expressed and are under the control of a downstream tissue-specific enhancer. The *GH2* locus encodes a protein that differs from the *GH1*-derived growth hormone at 13 amino acid residues. All five genes share a very similar structure with five exons interrupted at identical positions by short introns, 260bp, 209bp, 92bp and 253bp in length in the case of *GH1* (Figure 2).

Exon 1 of the *GH1* gene contains 60bp of 5' untranslated sequence (although an alternative transcriptional initiation site is present at -54), codons -26 to -24 and the first nucleotide of codon -23 corresponding to the start of the 26 amino acid leader sequence. Exon 2 encodes the rest of the leader peptide and the first 31 amino acids of mature GH. Exons 3-5 encode amino acids 32-71, 72-126 and 127-191, respectively. Exon 5 also encodes 112bp 3' untranslated sequence culminating in the polyadenylation site. An *Alu* repetitive sequence element is present 100bp 3' to the *GH1* polyadenylation site. Although the five related genes are highly homologous throughout their 5' flanking and coding regions, they diverge in their 3' flanking regions.

The *GH1* and *GH2* genes differ with respect to their mRNA splicing patterns. As noted above, in 9% of *GH1* transcripts, exon 2 is spliced to an alternative acceptor splice site 45bp into exon 3 to generate a 20 kDa isoform instead of the normal 22 kDa. The *GH2* gene is not alternatively spliced in this fashion. A third 17.5 kDa variant, which lacks the 40 amino acids encoded by exon 3 of *GH1*, has also been reported.

The *CSH1* and *CSH2* loci encode proteins of identical sequence and are 93% homologous to the *GH1* sequence at the DNA level. By comparison with the *CSH* gene sequences, the *CSHP1* pseudogene contains 25 nucleotide substitutions within its "exons" plus a G→A transition in the obligate +1 position of the donor splice site of intron 2 that partially inactivates its expression.

A number of biallelic restriction fragment length polymorphisms (RFLPs) have been reported within the GH gene region. Five of these (two *Bgl*II, two *Msp*I, one *Hinc*I) occur in Caucasians and Blacks whereas a further *BamH*I polymorphism occurs predominantly in Blacks. Strong linkage disequilibrium has been observed between these polymorphisms consistent with the relatively recent evolutionary origin of the gene cluster. The *Hinc*II and *BamH*I polymorphisms occur immediately 5' to the *GH1* gene. An *Rsa*I polymorphism occurs in the *GH1* promoter region resulting from an A/G dimorphism at nucleotide -75 whilst a relatively frequent *Sph1* polymorphism remains to be fully characterised. A highly informative (83% heterozygosity) variable number repeat polymorphism has been located some 19kb 3' to the *GH1* gene; formatted for PCR, the 18 distinct alleles of this polymorphism can be distinguished by fragment size (201 to 253bp).

Finally, the *GH1* gene promoter/5'-untranslated region has been found to exhibit a very high level of sequence polymorphism with 17 variant nucleotides within a 570 bp stretch (Table 2A):

**Table 2A:**

| **Known polymorphisms in the human *GH1* gene promoter/5' untranslated region [after Giordano *et al. Human Genetics* 100: 249-255 (1997) and Wagner et al. *Eur. J. Endocrinol*. 137: 474-481]. (Figure 3).** | |
|---|---|
| **Nucleotide location** | **Polymorphism (alternative nucleotides)** |
| -476 | G/A |
| -364 | G/T |
| -339 | ΔG |
| -308 | T/G |
| -301 | T/G |
| -278 | T/G |
| -272 to -276 | CCAGA/SMRRR |
| -168 | T/C |
| -75 | A/G |
| -57 | G/T |
| -31 | ΔG |
| -6 | G/A |
| - 1 | T/A/C |
| +3 | G/C |
| +16 | A/G |
| +26 | A/C |
| +59 | T/G |

These 17 variable positions can be ascribed to 23 different haplotypes in our patient population. The polymorphisms at positions -1, +3 and +59 are predicted to cause amino acid substitutions in the *GHDTA* protein, putatively encoded by this region of the *GH1* gene promoter (see below). Some of the sequence variants occur in the same positions in which the *GH1* gene differs from the other placentally-expressed genes suggesting that the mechanism might be gene conversion and that the placental genes have served as donors of the converted sequences.

In a study of prepubertal short children with GH insufficiency, Hasegawa *et al.* [*J*. *Clin. Endocrinol Metab* 85, 1290-1295 (2000)] reported an association between three polymorphisms in the *GH1* gene [IVS4 C →T 1101, T/G -278, T/G -57] and both GH secretion and height.

Since the first *GH1* gene deletions were reported, a variety of more subtle lesions have been described. In some cases, these lesions have been associated with unusual types of GH deficiency and are potentially important as a means of obtaining new insights into GH structure and function

**Table 2B:**

| **Gross deletions involving the *GH1* gene, or in the vicinity of the *GH1* gene, that cause GH deficiency and short stature** | | | |
|---|---|---|---|
| **Deletion size (kb)** | **Loci involved** | **Comments** | **Post-treatment antibodies present?** |
| 6.7 | *GH1* | Swiss family | yes |
| 6.7 | *GH1* | Japanese family | yes |
| 6.7 | *GH1* | Argentinan family of Spanish ancestry. Homozygous. | yes |
| 6.7 | *GH1* | Austrian family | yes |
| 6.7 | *GH1* | Brazilian family | yes |
| 6.7 | *GH1* | Patient with short stature and cystic fibrosis | yes |
| 6.7 | *GH1* | Various | no |
| 7.6 | *GH1* | Iraqi, Yemeni and Iranian families | no |
| 7.6 | *GH1* | Italian family. Homozygous. Consanguinous marriage | yes |
| 7.6 | *GH1* | Italian and Turkish families | yes |
| 7.6 | *GH1* | Spanish family | no |
| 7.6 | *GH1* | Various | yes |
| 7.0 | *GH1* | Canadian family | yes |
| 7.0 | *GH1* | Mexican family | yes |
| 7.0 | *GH1* | Chinese family. Homozygous | no - No treatment with GH. |
| *45* | *GH1*, *CSHP1*, *CSH1, GH2* | Turkish family, Homozygous. Consanguinous marriage | yes |
| 45 | *GH1, CSHP1*, *CSH1, GH2* | Italian family. Homozygous | yes |
| 45 | *GH1, CSHP1*, *CSH1, GH2* | Italian family. Homozygous. Consanguinous marriage | yes |
| 45 | *GH1, CSHP1, CSH1, GH2* | "Asian" family | no |
| ? | *CSH1, GH2, CSH2* | Italian family. Heterozygous | no |
| ? | *CSH1, GH2, CSH2* | Danish family. Compound heterozygous for non-identical deletions | no |
| Double | (i) *GH1* (6.7kb) | French origin (Romany). | yes |
| | (ii) *CSH1, GH2, CSH2* (∼32kb) | Homozygous. Consanguinous marriage. | |

The gene encoding growth hormone (*GH1*) was one of the first human genes to be cloned and the first gross gene deletions (6.7kb type) responsible for inherited growth hormone deficiency were soon detected by Southern blotting. All gross deletions involving the *GH1* gene result in severe (type IA) deficiency, characterised by the total absence of GH. ∼70% of characterised deletions of the *GH1* gene are 6.7 kb in length, whilst most of the remainder are of 7.6 kb or 7.0 kb (Table 2B).

In addition, several examples of much more infrequent deletions have been reported.In recent years, various attempts have been made to move away from Southern blotting toward PCR-based approaches as a mutation screening tool. Homozygous *GH1* gene deletions have been fairly readily detected by PCR amplification of the *GH1* gene and flanking regions followed by restriction enzyme digestion of the resulting PCR products. Although this approach has been used successfully to exclude homozygosity for a *GH1* gene deletion in at-risk pregnancies, it is however unable to distinguish homozygosity for the wild-type gene from heterozygosity for a gene deletion. It would also fail to detect deletions other than the relatively short 6.7, 7.0 and 7.6kb deletions that remove only the *GH1* gene.

PCR primers have been designed which immediately flank the *GH1* gene and which generate a 790bp fragment from control DNA samples. Absence of this fragment was held to be indicative of a *GH1* gene deletion but the use of "non-specific PCR fragments" as internal controls for PCR amplification must make the reliability of this method somewhat suspect.

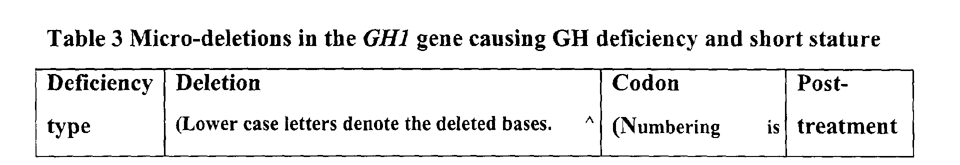

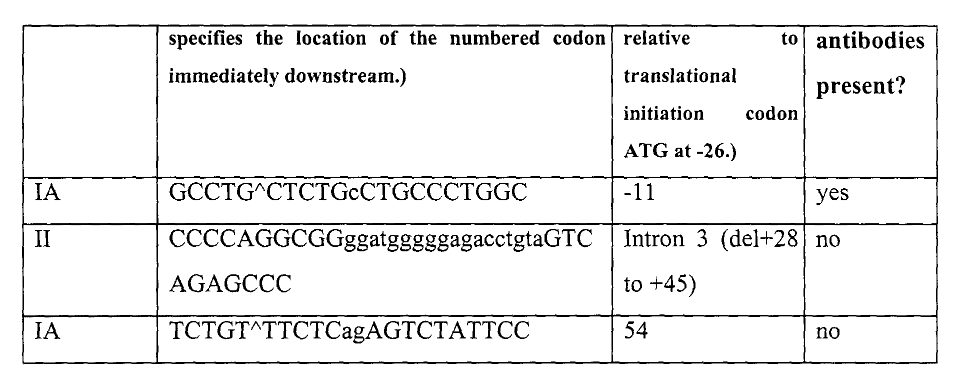

As well as gross deletions, three micro-deletions of the *GH1* gene have been reported; two of these patients were also heterozygous for the 6.7 kb *GH1* gene deletion (Table 3).

Only six different single base-pair substitutions have been reported from within the coding region of the *GH1* gene (Table 4). Two of these are nonsense mutations converting amino acid residues Trp-7 and Glu-4 in the signal peptide to stop codons. These mutations are the only known *GH1* gene lesions to cause type IA deficiency that are not gene deletions. Since these lesions predict termination of translation within the signal peptide, they would be incompatible with the production of a functional GH molecule. The other four single base-pair substitutions are missense mutations that result in the production of dysfunctional growth hormone molecules.

**Table 4**

| **Single base-pair substitutions in the *GH1* coding region causing GH deficiency and short stature** | | | | |
|---|---|---|---|---|
| **Deficiency type** | **Nucleotide substitution** | **Amino acid substitution** | **Codon (numbering relative to translational initiation codon ATG at -26)** | **Post-treatment antibodies present?** |
| IA | ACA→GCA | Thr→Ala | -24 | no |
| IA | TGG→TAG | Trp→Term | -7 | no |
| IA | GAG→TAG | Glu→Term | -4 | yes |
| II | CGC→TGC | Arg→Cys | 77 | no |
| ? | GAC→GGC | Asp→Gly | 112 | no |
| ? | CGC→CAC | Arg→His | 183 | no |

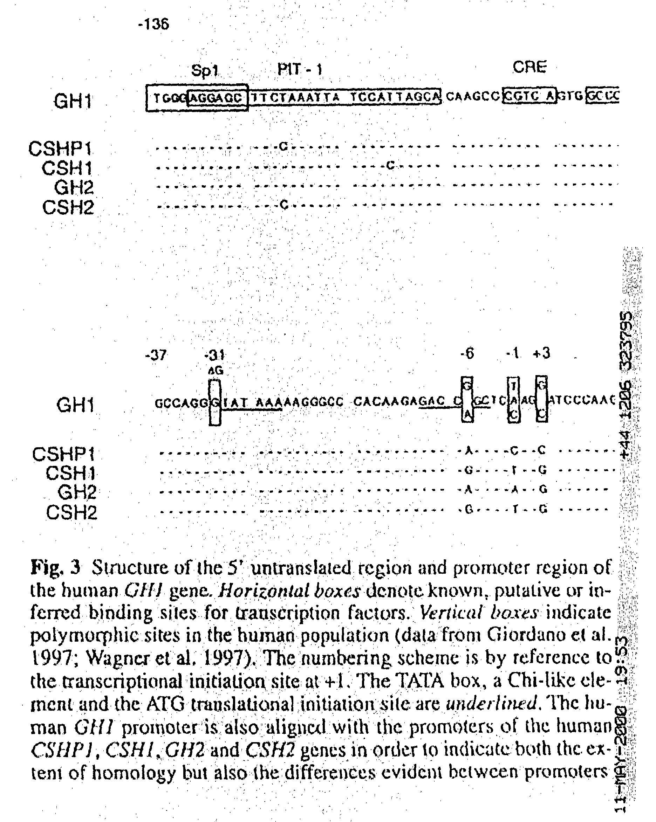

Single base-pair substitutions in the promoter region of possible pathological significance were first sought by sequencing the promoter region of the *GH1* gene (between -60 and +70 relative to the transcriptional initiation site) in three Chinese patients with IGHD IA and 2 controls. Several differences were noted but these were probable polymorphisms and were not characterised further. As mentioned above, the promoter region of the *GH1* gene has subsequently been shown to exhibit a very high level of sequence polymorphism with 17 variant nucleotides within a 570 bp stretch (Figure 3). However, they were not found to be over-represented in patients as compared to controls.

*GH1* promoter variation has also been separately investigated and a total of 22 variant polymorphic sites were detected, mostly single base-pair substitutions: 17 of these occurred in a 550 bp region 5' to the ATG initiation codon, three occurred around position -1075 5' to ATG, and two occurred within intron 1 (IVS1) at positions 76 and 219 respectively [Wagner *et al*, Eur J Endocrinol 137 474-81 (1997)]. All except four of these variants were also noted in controls but these four variants were not considered to be the cause of the growth hormone deficiency. Only one of the variant sites occurred within a sequence homologous to a transcription factor binding site: the alternative presence of CCAGA and GAGAG sequences at -333 within a potential (but not proven) NF-1 binding site.

Therefore, to date, no mutations of pathological significance have been reported in the *GH1* gene promoter.

Single base-pair substitutions affecting mRNA splicing have also been described in the *GH1* gene. Most are associated with a comparatively rare dominant form of GH deficiency (Table 5).

The transversions in the intron 4 donor splice site have been shown by mRNA *in vitro* expression analysis of transfected cells to activate a cryptic splice site within exon 4, 73bp 5' to the exon 4 donor splice site. This would predict the generation of an aberrantly spliced product lacking amino acids 103-126 encoded by exon 4 and, as a consequence of a shift in the reading frame, the incorporation of 94 novel amino acids including 29 resulting from read-through of the normally untranslated 3' non-coding region of the *GH1* gene.

**Table 5**

| **Single base-pair substitutions affecting mRNA splicing and causing GH deficiency and short stature** | | | |
|---|---|---|---|
| **Deficiency type** | **Nucleotide substitution/ position** | **Splice site** | **Ethno-geographic origin/zygosity** |
| II | G→A, +1 | IVS3 donor | Sweden, North America, Northern Europe, South Africa, Chile/heterozygous |
| II | G→C, +1 | IVS3 donor | Turkish/ heterozygous |
| II | G→A, +5 | IVS3 donor | Chilean/ heterozygous |
| II | G→C, +5 | IVS3 donor | ? |
| II | T→C, +6 | IVS3 donor | Turkish/ heterozygous Asian/ heterozygous |
| II | G→A, +28 | IVS3 donor | ?/heterozygous |
| IB | G→C, +1 | IVS4 donor | Saudi Arabian/ homozygous |
| IB | G→T, +1 | IVS4 donor | Saudi Arabian/ homozygous |
| IB | G→C,+5 | IVS4 donor | ? |

Since the region of the GH protein encoded by exons 4 and 5 is thought to be important for correct targeting of the protein to secretory granules, it has been predicted that this aberrant protein would not be secreted normally. However, no antibodies to exogenous GH have been noted in patients with type IB GH deficiency. The avoidance of immune intolerance may thus indicate that at least some of the aberrant protein product could be secreted and that it could be partially stable in the circulation. By contrast, the five known splicing mutations within IVS3 (Table 5) are associated with a type II deficiency state manifesting autosomal dominant inheritance through the affected families.

GH deficiency patients with truncating *GH1* mutations or homozygous gene deletions are at considerable risk of developing anti-GH antibodies upon GH treatment. By contrast, we are not aware of any reports describing allo-antibody formation in patients with either missense mutations or single base-pair substitutions within splice sites.

Until now, no other correlations between mutant genotype and clinical phenotype have been reported. The requisite data in the published literature are sparse and very variable in quality, but we have attempted a crude meta-analysis as a means of gauging whether or not patients with gross gene deletions differ from patients with splice site mutations in terms of their clinical and phenotypic sequelae. The height of the patients with *GH1* deletions was found to be on average 7.3 SD below the age-adjusted mean (n=29), as compared with an average of 5.4 SD below the mean (n=17) for the patients with *GH1* splicing mutations. Although bone age delay was greater and growth velocity lower in the deletion patients, such findings are very difficult to interpret since they may be subject to bias of ascertainment.

Since most cases of familial GH deficiency hitherto described are inherited as an autosomal recessive trait, some examples of the inherited deficiency state are likely to have gone unrecognized owing to small family size. Similarly, cases of GH deficiency resulting from *de novo* mutations of the *GH1* gene could be classified as sporadic, and a genetic explanation for the disorder would neither be entertained nor sought. Finally, depending upon the criteria used for defining the deficiency state, it may be that the full breadth of both the phenotypic and genotypic spectrum of GH deficiency may never have come to clinical attention. For these reasons, current estimates of the prevalence of GH deficiency could be inaccurate and may therefore seriously underestimate the true prevalence in the population.

The definition of IGHD favoured by many combines (a) severe growth retardation, often - as mentioned above - defined as <-4.5 SD in height; (b) reduced GH response to stimulation/provocation (*ie* a serum GH level of <4ng/ml); and (iii) no other cause for growth retardation. The strict adherence to formal definitions of what constitutes GH deficiency and the fairly uniform acceptance of these criteria, especially criterion (b) in selecting patients for study [Shalet SM *et al. Endocrine Rev.* 19: 203-223 (1998)] would have served to ensure that the described *GH1* mutational spectrum was not only far from complete but also unrepresentative of the wider mutational spectrum. Thus, mutations responsible for GH deficiency states in which the SD scores were less severe or the GH levels less reduced (*eg* missense mutations within the coding region of the gene or promoter mutations) would have been much less likely to come to clinical attention. Indeed, this may go some way toward explaining why only four different missense mutations have so far been reported in the *GH1* gene, a finding which is virtually unprecedented for a fairly prevalent disorder that has been studied at the molecular level for nearly 20 years [Krawczak *et al*, Hum. Mutation 15, 45-51 (2000)].

The complete absence of GH produces a readily recognisable and severe clinical phenotype that has been extensively studied. In those reported studies in which the phenotype of the patients is less severe and in which patient selection criteria have actually been identified, patient ascertainment strategies have generally used the deviation of an individual's height from the mean height for their age as a diagnostic indicator of growth failure.

The selection of patients using criteria (a) and (b), as defined above, will serve to define patients with a severe degree of IGHD-related growth failure. We have proposed that moderating the criteria applied in selecting patients for study would be likely to lead to the inclusion of patients whose growth failure is a manifestation of a different portion of the GH deficiency spectrum, and which could therefore yield a novel set of underlying mutational lesions. Some of these novel lesions could give rise to stable, yet dysfunctional, GH molecules that would exhibit normal immunological reactivity but little or no biological activity. On the basis of radioimmunoassay test results, dysfunctional GH molecules would have been erroneously regarded as normal. If such dysfunctional variants were to turn out to be common, then it would follow that GH deficiency is being under-diagnosed as a result of our current dependence on radio-immunoassay-based GH "function tests". Further, it would demonstrate an urgent need for the development of a true functional diagnostic assay.

We believe that height velocity is a more sensitive indicator of growth failure than absolute height measurements. The use of height velocity in conjunction with an assessment of bone age delay (retarded osseus maturation also due to GH deficiency); and other variables being normal, has allowed us to identify a unified group of patients with phenotypes which are less severe than that of classical IGHD patients having no GH, but who are more likely to have lesions of the *GH1* gene than those selected on the basis of height measurements alone.

Accordingly, the present invention provides a detection method for detecting a variation in *GH1* effective to act as an indicator of GH dysfunction in a patient, which detection method comprises the steps of:
(a) obtaining a test sample comprising a nucleotide sequence of the human *GH1* gene from the patient; and
(b) comparing the sequence obtained from the test sample with the standard sequence known to be that of the human *GH1* gene, wherein a difference between the test sample sequence and the standard sequence indicates the presence of a variation effective to act as an indicator of GH dysfunction (hereinafter "GH variant") characterised in that the test sample is obtained from a patient exhibiting the following cumulative criteria:
   (i) height velocity below the 25^{th} centile for age;
   (ii) bone age delay according to the Tanner-Whitehouse scale of at least two years, when compared with chronological age; and
   (iii) no other disorder known to cause inclusion in criteria (i) to (ii) above.

The present invention therefore further provides a GH variant detected by or detectable according to the above-described method of this invention.

With respect to the cumulative criteria (i) to (iii), each criterion may be assessed according to known methods and parameters readily available and described in the art, as elaborated further below:
(i) Tanner JM, Whitehouse RH. *Atlas of Children's Growth.* London: Academic Press, 1982; and Butler *et al. Ann. Hum. Biol.* 1990; 17: 177-198 (1990) are sources for statistics enabling a determination of the first criterion, *viz* that the height velocity of the patient is less than the 25^{th} centile for the patient's age.
(ii) The Tanner-Whitehouse scale for assessing years of bone age delay is described by Tanner JM, Whitehouse RH, Cameron N *et al* in *Assessment of Skeletal Maturity and Prediction of Adult Height*. London: Academic Press, 1983.
(iii) Since short stature may also be secondary to conditions other than GH dysfunction, test samples from patients suffering from such disorders are excluded from the method of the invention. That the patient is suffering from no other disorder that might give rise to similar symptoms to that of GH dysfunction is determined by baseline investigations. "Baseline investigations" therefore include tests to exclude, particularly, hypothyroidism; pseudo-hypoparathyroidism; malabsorption syndromes *eg* coeliac disease; renal and hepatic diseases; haematological disorders, such as anaemia; and a karyotype to check that a chromosome disorder such as Turner syndrome is not the cause of the growth failure. The patient may also have had a thorough clinical examination in order to exclude other causes of growth failure, for example, cardiac disease including congenital heart disease; chronic auto-immune conditions, such as rheumatoid arthritis and inflammatory bowel disease; chronic respiratory conditions, such as severe asthma or cystic fibrosis; and skeletal problems, such as achondroplasia. A full medical history will also have been taken and used to complement the medical examination in order to aid the exclusion not only of the physical disorders identified above but also of psycho-social deprivation, another well-recognised cause of growth failure in childhood.
   Optionally, the patient may also have been subjected to one or more growth hormone function tests. The term "growth hormone function tests" refers to tests of growth hormone secretion, such as those stimulation tests mentioned herinbefore, particularly the insulin-induced hypoglycaemic test (IST).
   GH function tests are usually carried out on patients who are short; have been clinically assessed and had their height monitored over more than one visit to the endocrine clinic; have no other detectable cause for their growth failure; and therefore warrant being subjected to an assessment of their ability to produce growth hormone secretion from their pituitary gland following an appropriate stimulus, such as the profound drop in blood glucose that results from the administration of intravenous insulin.
   Preferably, in the selection criteria for use in the detection method of this invention, a further criterion may also be applied in addition to (i) to (iii) above, namely:
(iv) growth failure, defined as a growth pattern [delineated by a series of height measurements; Brook CDG, Ed., *Clinical Paediatric Endocrinology,* 3rd Ed., (1995) Blackwell Science. Chapter 9, p141] which, when plotted on a standard height chart [Tanner *et al. Arch. Dis. Child* 45: 755-762 (1970)], predicts an adult height for the patient which is outside the patient's estimated target adult height range, the estimate being based upon the heights of the patient's parents.

Also useful as a reference is Tanner and Whitehouse. *Arch. Dis. Child* 51: 170-179 (1976)]. A patient's target adult height range is calculated as the mid-parental height (MPH) with the range being the 10th to 90th centile for MPH, which is sex-dependent:
MPH if male = [father's height + (mother's height +13)]/2 + or - in the range of from 6 to 8cm, usually 7.5cm; and
MPH if female = [(father's height - 13) + mother's height]/2 + or - in the range of from 6 to 8 cm, usually 6cm

These are standard tests and measurements used in the field and any other acceptable method of calculation can be used to determine growth failure, although the above-described method based on the description in Brook (*ibid*, 1996) regarding the formula to apply for predicting the limits of the target height range and on the description in Tanner (*ibid*, 1970) regarding the standard height charts are preferred according to this invention.

In the detection method according to this invention, therefore, although current height may be measured in order to apply the above-noted criteria, this is not in itself a criterion used for selection of patients in this method. As mentioned above, prior art methods rely on standard deviation from 'normal' height (*ie* absolute growth) as the criterion for selecting patients. The present invention does not require inclusion of such criterion and therefore the present invention provides a detection method in which absolute height is or may be excluded as a selection criterion.
Increasing the breadth of the *GH1* mutational spectrum will inevitably lead to a redefinition of inherited GH deficiency in molecular genetic terms. Furthermore, the recognition of novel types of short stature must eventually require the reclassification of GH deficiency as a disease entity. This will obviously have important implications for the screening and identification of individuals with short stature in whom the use of growth hormone treatment might be beneficial.

The test sample obtained from the patient in the detection method of the invention preferably comprises genomic DNA extracted from patient lymphocytes by standard procedures. *GH1* gene analysis is thereafter preferably carried out according to the following steps:
1(a). PCR amplification of a 3.2 kb fragment containing the *GH1* gene in its entirety (promoter, five exons of the coding region, introns and untranslated regions) followed by the nested PCR of smaller, overlapping constituent fragments using primers designed so as to ensure *GH1* gene specificity. As well as using six known primers, the design of novel *GH1*-specific primers was found to be essential in order to avoid cross-contamination emanating from inadvertent PCR amplification of the paralogous, closely linked and highly homologous *GH2, CSH1* and *CSH2* gense, and the *CSHP1* pseudo-gene; and/or
1(b). PCR amplification of all or a fragment of genomic DNA spanning the Locus Control Region (hypersensitive sites I and II) approximately 15 kb upstream of the *GH1* gene of the patient [Jones *et al* Mol Cell Biol 15 7010-21 (1995)]. The Locus Control Region (LCR) is an enhancer region that affects the level and time of *GHT* transcription. The LCR is located ∼14 kb 5' to the *GH1* gene and is responsible for the coordinate expression of the genes in the GH gene cluster. PCR amplification was carried out, using novel oligonucleotide primers, on two overlapping fragments (254 bp and 258 bp) in all patients;
2. Mutational screening of the *GH1* gene fragments by Denaturing High Performance Liquid Chromatography (DHPLC) using the Transgenomic WAVE ™System [O'Donovan *et al. Genomics* 52: 44-49 (1998)]. This screening method was selected for use since it is extremely rapid, cheap, sensitive and reproducible and exhibits, at least in our hands, a detection efficiency >95%. "Bandshifts" detected by DHPLC would represent potential DNA sequence variants; and
3. Characterisation of any such variants by DNA sequencing (either by automated or manual methods); and, optionally, but preferably also
4. Functional characterisation of *GH1* gene lesions using methodology appropriate to the location of the lesion and the inferred mechanism of dysfunction.

Therefore, the present invention further provides novel *GH1*-specific primers for use in the analysis of *GH1* as described above and in the examples, which primers include:
novel primers suitable for use in the DHPLC step (see Example 3, Table 6, for further details): 
CTC CGC GTT CAG GTT GGC (GHD1F);
AGG TGA GCT GTC CAC AGG (GHD1R);
CTT CCA GGG ACC AGG AGC (GHD2R);
CAT GTA AGC CAA GTA TTT GGC C (GHD3F);
GGA GAA GGC ATC CAC TCA CGG (GHD4R);
TCA GAG TCT ATT CCG ACA CCC (GHD5F);
CGT AGT TCT TGA GTA GTG CGT CAT CG (GHD6R); and
TTC AAG CAG ACC TAC AGC AAG TTC G (GHD7F);
 and primers suitable for use in the LCR step (all 5'→3', see also Example 5):

GTGCCCCAAGCCTTTCCC (LCR15: 1159-1177);
TGTCAGATGTTCAGTTCATGG (LCR13: 1391-1412);
CCTCAAGCTGACCTCAGG (LCR25: 1346-1363); and
GATCTTGGCCTAGGCCTCG (LCR23: 1584-1602).

The detection method of the invention and the GH variant identifiable or detectable thereby can give rise to the following additional advantages:
1. Expansion of the known spectrum of *GH1* gene mutations by identification and characterisation of new lesions.
2. Evaluation of the role of *GH1* gene mutations in the aetiology of short stature.
3. Identification of the mode of inheritance of novel *GH1* gene lesions.
4. Elucidation of the relationship between mutant genotype and clinical phenotype. This is deemed essential for the early detection and appropriate clinical management of GH deficiency.
5. Evaluation of the effects of *GH1* mutations on the structure and function of the GH molecule. This is particularly important for the assessment of those children with a clinical phenotype at the milder end of the clinical spectrum of short stature. In this group of patients, dysfunctional GH may be produced that is immunologically active and therefore falls within the normal range in GH function tests.
6. Development of rapid DNA diagnostic tests for inherited GH deficiency
7. Assessment of our postulate that GH deficiency is currently underdiagnosed and underestimated in the population.

Therefore, the characterisation of further, naturally occurring *GH1* lesions promises to be of considerable importance to studies of GH structure, function and expression. Studies of novel coding sequence variants should increase our understanding not only of GH function, but also of the interactions between GH and its receptor (GHR), and the process of GHR-mediated signal transduction. Insights obtained could be relevant to the rational design of a new generation of therapeutic agents. Similarly, studies of naturally-occurring *GH1* lesions in the promoter region should provide new insights into the control of *GH1* gene expression. Thus it may be seen that a broad spectrum of mutational lesions will necessarily improve our understanding of the relationship between mutant genotype and clinical phenotype in inherited forms of GH deficiency. Clearly, these studies are essential for the early detection and appropriate clinical management of familial GH deficiency.

The present invention therefore further provides a variant of *GH1*, which differs from *GH1* and is detectable by the method according to the invention but is not detectable by methods used hitherto, such as those reliant on patient selection criteria based primarily on height. Such *GH1* variants of the invention include those characterised in Example 6 and Table 7 hereinafter.

As indicated hereinbefore, current tests to assess GH secretion are many and varied and no single currently available investigation is ideal. Since the secretion of human GH is pulsatile, and because the amplitude and frequency of the GH pulses are extremely variable (being influenced by multiple internal and external factors including sleep, exercise, stress and the pubertal stage of the individual concerned), those tests that yield the best information require close supervision of the patient in a dedicated investigation ward. The tests are therefore time-consuming, expensive, and cause considerable stress and distress to the patient and their family. The insulin-induced hypoglycaemic test (IST) is of particular note; it is used by many doctors, as mentioned above, to assess GH secretion but deaths have occurred owing to the treatment necessary for the hypoglycaemia induced in the patient as a necessary requirement of its successful implementation. It is therefore of paramount importance that the decision to perform an investigation, such as an IST, is most carefully considered before it is given a place in the assessment of a short child. The development of a DNA test for use in screening short patients would therefore have many advantages over the other tests currently available.

Accordingly, the present invention provides a screening method for screening a patient suspected of having dysfunctional GH, which screening method comprises the steps of:
(a) obtaining a test sample comprising a nucleotide sequence of the human *GH1* gene from the patient; and
(b) comparing a region of the sequence obtained from the test sample with the corresponding region of a predetermined sequence characterised in that the predetermined sequence is selected from a GH variant detectable according to the above-described method of the present invention.

More specifically, the screening method of the invention is characterised in that the predetermined sequence is an oligonucleotide having a nucleic acid sequence corresponding to a region of a variant GH1 gene, which region incorporates at least one variation when compared with the corresponding region of the wild type sequence.

Especially preferred is when the variation is one detectable by the detection method of the invention, such as any of those identified in Example 6 and Table 7 hereinafter.

Preferably, the test sample comprises genomic DNA, which may be extracted by conventional methods.

Therefore, the present invention further provides a screening method for determining GH dysfunction, comprising:
(a) obtaining a first human tissue suspected of GH dysfunction; and
(b) comparing the *GH1* gene or *GH1* transcript, or fragment therefrom (*eg* cDNA), in the first human tissue to the corresponding gene, transcript or fragment of a *GH1* variant obtainable from a second human tissue derived from a human exhibiting the following cumulative criteria:
   (i) height velocity below the 25^{th} centile for age;
   (ii) bone age delay according to the Tanner-Whitehouse scale of at least two years, when compared with chronological age; and
   (iii) no other disorder known to cause inclusion in criteria (i) to (ii) above; and, optionally,
   (iv) growth failure defined as a growth pattern [delineated by a series of height measurements; Brook CDG, Ed., *Clinical Paediatric Endocrinology*, 3rd Ed., (1996) Blackwell Science. Chapter 9, p141] which, when plotted on a standard height chart [Tanner *et al. Arch. Dis. Child* 45: 755-762 (1970)], predicts an adult height for the patient which is outside the patient's estimated target adult height range, the estimate being based upon the heights of the patient's parents.

In the screening method of the invention, the comparison step may be carried out in conventional manner, for example by sequencing the appropriate region of the *GH1* gene, particularly in the case where relatively few variants are to be detected/compared. Where relatively large numbers of variants are involved, DNA chip technology may be employed, wherein the chip is a miniature parallel analytical device that is used to screen simultaneously for multiple mutations by hybridisation of labelled sample DNA (cDNA or genomic DNA derived from the patient) to microarrays of mutation-specific oligonucleotide probes immobilised on a solid support [Southern, *Trends Genet*. 12: 110-115 (1996)].

The advantage of a DNA screening method according to the invention over current tests include:
1. It involves, for the patient, only a single blood test that can be performed in a clinic. Hospital admission, prolonged medical supervision and repeated blood sampling would not be required as is the case for the majority of currently-available tests. There would therefore be a reduction in the expense incurred, the use of specialist time and the distress caused for each patient tested.
2. Earlier diagnosis of functional GH deficiency in patients would become possible. The ease with which the DNA screen can be performed would allow the clinician to consider such an investigation much earlier in the management of a patient than might otherwise be the case. Currently, owing to the problems inherent in tests for GH secretion, doctors will assess children in the out-patient clinic over a long period of time, sometimes several years, before they will subject a child to an 1ST. The early diagnosis of a genetic aetiology for GH deficiency would enable earlier treatment with GH thereby bringing forward the opportunity to treat patients appropriately by months, or even years in individuals with a less severe phenotype.
3. More patients could be tested for GH dysfunction. The ease of the DNA test would allow the doctor to perform it as part of the initial assessment of all short patients at their first visit to the endocrine clinic. This is likely to reveal patients with lesions of the *GH1* gene that cause severe growth problems and also those with milder lesions (e.g. missense mutations in the coding region). These patients may not previously have come to clinical attention because their clinical/phenotypic problems would not have been severe enough to warrant an IST, but they might nevertheless still benefit from treatment with GH.
4. Early identification of patients who will require life-long treatment with GH would be possible. These patients could be identified and treated appropriately without recourse to either initial testing or re-testing for GH secretion, or the use of a period without GH to assess their progress (a "trial without treatment").
5. Easy and early identification of family members with GH dysfunction would become available. Once the genetic lesion responsible for growth problems has been identified in an individual, it is relatively easy to assess other family members for the same genetic lesion and to ascertain whether they would also gain benefit from treatment with GH.
6. Accuracy of diagnosis should increase. Tests for GH secretion are notorious for their variability in terms of reproducibility of assay results, both within and between laboratories. DNA screening would make this problem a thing of the past. In addition, GH secretion test results can be very difficult to interpret in certain situations, for example, if the patient is also hypothyroid or has delayed puberty. DNA screening would remove this doubt and prevent delay in the initiation of GH treatment for those patients in whom its use would be beneficial

Accordingly, the present invention further provides a kit suitable for use in carrying out the screening method of the invention, which kit comprises:
(a) an oligonucleotide having a nucleic acid sequence corresponding to a region of a variant *GH1* gene, which region incorporates at least one variation from the corresponding wild-type sequence; and
(b) an oligonucleotide having a nucleic acid sequence corresponding to the wild-type sequence in the region specified in (a); and, optionally,
(c) one or more reagents suitable for carrying out PCR for amplifying desired regions of the patient's DNA.

Such reagents may include, for example, PCR primers corresponding to the exon of the *GH1* gene, and/or primers mentioned herein, especially novel primers mentioned hereinabove; and/or other reagents for use in PCR, such as *Taq* DNA polymerase.

Preferably, the oligonucleotides in the kit comprise in the range of from 20 to 25 base-pairs, such as 20 base-pairs for the variant sequences and either 20 for the wild-type in the case where the variant is a single base-pair substitution or 25 base-pairs where the variant is a 5 base-pair deletion. In any case, the oligonucleotides must be selected so as to be unique for the region selected and not repeated elsewhere in the genome.

Obviously, in the situation where it is desired to screen for multiple variations, such as in the range of from 15 to 20 or more, this would necessitate a kit comprising up to 40 oligonucleotides or more. In the alternative screening method, therefore, using DNA chip technology, the present invention provides a plurality of oligonucleotides as defined in kit component (a) above immobilised on a solid support.
*GH1* variants detectable by the detection method of this invention may have additional uses than as standards in a screening test for GH dysfunction. For example, variants other than those where the variation is in the promoter region of the *GH1* gene may be used to treat a patient wherein GH production is over-stimulated, such as in cases of pituitary gigantism or acromegaly.

Accordingly, the present invention further provides a composition comprising a variant of *GH1*, especially a variant detectable by the detection method of this invention and identified herein, in association with a pharmaceutically acceptable carrier therefor.

Furthermore, the invention provides:
(a) a nucleic acid sequence encoding a variant of *GH1*
(b) a sequence substantially homologous to or that hybridises to sequence (a) under stringent conditions; or
(c) a sequence substantially homologous to or that hybridises under stringent conditions to the sequence (a) or (b) but for the degeneracy of the genetic code; or
(d) an oligonucleotide specific for any of the sequences (a), (b) or (c).

Also provided are:
(a) a vector comprising the nucleic acid sequence described above;
(b) a host cell comprising the vector (a), such as a bacterial host cell; and
(c) a process for preparing a variant of *GH1*, which process comprises:
   (i) culturing the host cell (b); and
   (ii) recovering from the culture medium the variant of *GH1* thereby produced.

The present invention will now be illustrated with reference to the following Examples.

### Example 1 - Patient Selection

### Sources of Patients

Children with short stature have been identified through referral to the Regional Paediatric Growth, Endocrine and Diabetes Service at the University of Wales College of Medicine in Cardiff and by collaboration with other similar UK centres (*viz.* Newport, Birmingham, Bristol, Wrexham, Liverpool, Stoke-on-Trent, Portsmouth and Southampton). A full clinical history has been taken including family history, pedigree, documentation of growth parameters and previously-performed endocrine investigations. Accurate auxology was recorded wherever possible for the index case, parents and siblings. Blood samples for molecular genetic analysis were taken from the index case and appropriate close relatives. Further families were referred by Professor John A. Phillips III (Nashville, TN, USA), Dr Mohamad Maghnie (Pavia, Italy) and Dr Tamas Niederland (Gyor, Hungary). To date, samples from 69 GH-deficient families have been collected. Criteria used for the selection of patient 1 were:
Height velocity <25^{th} centile
Bone age delay of 3.5-4 years when compared with chronological age
Growth well below lower limit of % target height range
Growth hormone secretion tests normal
All other investigations normal

### Example 2 - Polymerase chain reaction (PCR) amplification of a GH1-specific fragment

PCR amplification of a 3.2 kb *GH1*-specific fragment has been performed on 65 unrelated patients. Genomic DNA was extracted from patient lymphocytes by standard procedures.

Oligonucleotide primers GH1F (5' GGGAGCCCCAGCAATGC 3'; -615 to -599) and GH1R (5' TGTAGGAAGTCTGGGGTGC 3'; +2598 to +2616) were designed to correspond to *GH1*-specific sequences in order to PCR amplify a 3.2kb single genomic DNA fragment containing the human *GH1* gene using the Expand™ high fidelity system (Roche).

Two separate thin-walled 0.65ml PCR tubes were used for each reaction. The first tube contained 500 nanograms (ng) each primer (GH1F and GH1R), 200µM dATP, dTTP, dCTP and dGTP and 200ng of patient genomic DNA made up to a final volume of 25µl with sterile water. The second tube contained 5µl 10x reaction buffer made up to a final volume of 24.25µl with sterile water. Both tubes were placed on ice for 5 minutes. After this time, 0.75µl of Expand™ polymerase mix was added to the second tube, the contents mixed and transferred to the first tube. The tube was centrifuged for 30 seconds and the reaction mixture overlaid with 30µl light mineral oil (Sigma). The reaction mixture was then placed in a 480 PCR programmable thermal cycler (Perkin Elmer) set at 95°C. The reaction mix was then amplified under the following conditions: 95°C for 2 minutes followed by 30 cycles of 95°C for 30 seconds, 58°C for 30 seconds and 68°C for 2 minutes. For the last 20 cycles, the elongation step at 68°C was increased by 5 seconds per cycle. This was followed by a further incubation at 68°C for 7 minutes and the reaction was then cooled to 4°C prior to further analysis. For each set of reactions, a blank (negative control) was also set up. The blank reaction contained all reagents apart from genomic DNA and was used to ensure that none of the reagents were contaminated.

A one-tenth volume (5µl) was analysed on a 1.5% agarose gel to assess whether PCR amplification had been successful before nested PCR was performed. Those samples that had PCR-amplified successfully were then diluted 1 in 100 prior to use for nested PCR.

### Example 3 - Nested-PCR

Nested PCR was performed on the fragments produced in Example 2 to generate, in each case, seven overlapping sub-fragments that together span the entire *GH1* gene. In addition, the Locus Control Region has been PCR-amplified (see Example 5) in all but three patients.

The seven overlapping sub-fragments of the initial 3.2 kb PCR product were PCR-amplified using *Taq* Gold DNA polymerase (Perkin-Elmer). Oligonucleotides used for these reactions are listed in Table 1 together with their sequence locations as determined from the *GH1* gene reference sequence.

A 1µl aliquot of the diluted long (3.2 kb) PCR product was put into a thin-walled 0.2ml PCR tube or into one well of a 96-well microtitre plate. To this was added 5µl 10x reaction buffer, 500ng appropriate primer pair (e.g. GH1DF and GH1DR), dATP, dTTP, dCTP and dGTP to a final concentration of 200µM, sterile water to a volume of 49.8µl, followed by 0.2µl *Taq* Gold polymerase.

The tube or microtitre plate was then placed in a Primus 96 thermal cycler (MWG Biotech) and cycled as follows: 12 min 95°C followed by 32 cycles of 95°C for 30 seconds, 58°C for 30 seconds and 72°C for 2 minutes. This was followed by further incubation at 72°C for 10 minutes and the reaction was then cooled to 4°C prior to further analysis.

A one-tenth volume (5µl) of the reaction mix was analysed on a 0.8% agarose gel to determine that the reaction had worked before denaturing high-pressure liquid chromatography (DHPLC) was performed on a WAVE™ DNA fragment analysis system (Transgenomic Inc. Crewe, Cheshire, UK). To enhance heteroduplex formation, the PCR product was denatured at 95°C for 5 minutes, followed by gradual re-annealing to 50°C over 45 minutes. Products were loaded on a DNAsep column (Transgenomic Inc.) and eluted with a linear acetonitrile (BDH Merck) gradient of 2%/min in a 0.1M triethylamine acetate buffer (TEAA pH 7.0), at a constant flow rate of 0.9ml/minute. The start and end points of the gradient were adjusted according to the size of the PCR product. Analysis took 6.5-8.5 minutes per amplified sample, including the time required for column regeneration and equilibration. Samples were analysed at the Melt temperatures (TM) determined using the DHPLCMelt software (http://insertion.stanford.edu/melt.html) and listed in Table 6. Eluted DNA fragments were detected by an UV-C detector (Transgenomic Inc.).

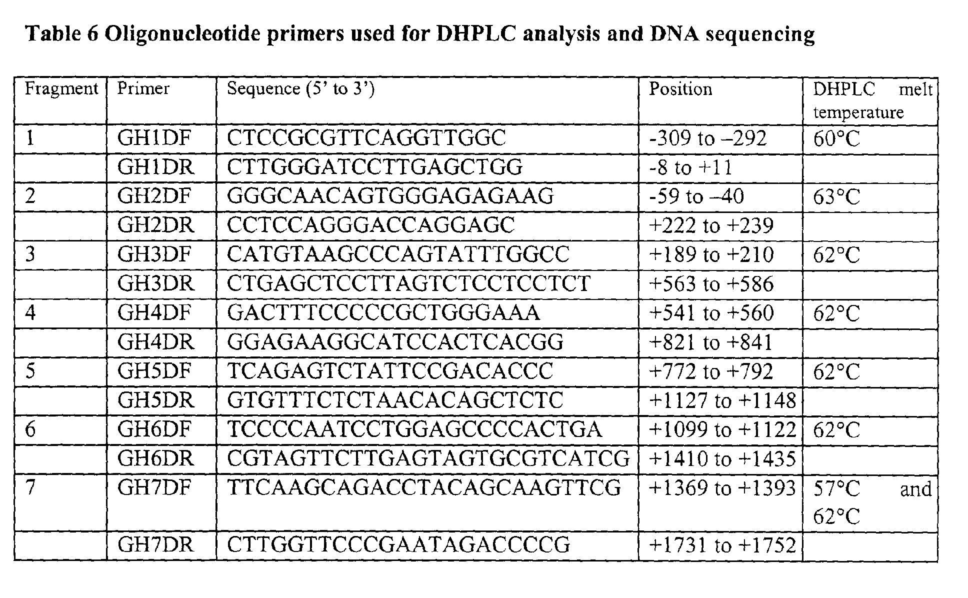

### Example 4 - Cloning and DNA-Sequencing of GH1-specific long PCR fragments

### Cloning

DHPLC analysis allowed the identification of DNA fragments containing putative DNA sequence changes. To determine which allele possessed the putative sequence change, *GH1*-specific long (3.2 kb) PCR fragments were cloned into the PCR plasmid cloning vector pGEM-T (Promega). Cloning was accomplished by adding 50ng of *GH1*-specific long PCR fragment to 10ng pGEM-T in the presence of 1x reaction buffer and 1µl (3 units) T4 DNA ligase in a final volume of 10µl. The reactions were incubated for 16 hours at 10°C. The entire reaction mixture was placed in a 1.5ml tube and cooled on ice. 50µl DH5α competent cells (Life Technologies) were added and the tube left on ice for 30 minutes. The mixture was then heat-shocked for 20 seconds at 37°C and returned to ice for 2 minutes. After this time, 0.95ml of YTx2 medium (16g tryptone, 10g yeast extract, 5g NaCl per litre water) was added and the mixture incubated at 37°C for one hour with shaking. The mixture was then plated out onto pre-warmed agar plates containing 50µg/ml ampicillin, IPTG and X-gal and incubated at 37°C for 16 hours to allow single colonies to grow.

Eight white colonies from each plate were picked and transferred to a second gridded plate. A small amount of each bacterial colony was PCR-amplified using primers GH1DF and GH1DR (see Example 3, Table 6) and the conditions previously described to determine that the *GH1*-specific long PCR fragment had been successfully cloned.

Clones that contained the *GH1* -specific long PCR fragment were grown in 2ml YTx2 medium; plasmid DNA was extracted from the bacteria using a Qiagen spin miniprep kit according to the manufacturer's instructions. DNA extracted in this way was quantified by measuring its optical density at 260nm and electrophoresed on a 0.8% agarose gel to verify that the size of the clone was correct. Four of these clones were then sequenced.

### Automated DNA sequencing

Clones containing the *GH1*-specific long PCR fragment were sequenced with the BigDye sequencing kit (Perkin Elmer) in either 0.2ml tubes or 96-well microtitre plates in a Primus 96 PCR thermal cycler. Oligonucleotide primers used for sequencing were:

GH1S1 (5' GTGGTCAGTGTTGGAACTGC 3': -556 to -537);
GH3DF (5' CATGTAAGCCAAGTATTTGGCC 3': +189 to +210);
GH4DF (5' GACTTTCCCCCGCTGTAAATAAG 3': +541 to +560): and
GH6DF (5' TCCCCAATCCTGGAGCCCCACTGA 3': +1099 to +1122).

1µg of cloned DNA was sequenced with 3.2pmol of the appropriate primer and 4µl BigDye sequencing mix in a final volume of 20µl. The tube or microtitre plate was then placed in the thermal cycler and cycled as follows: 2 minutes 96°C followed by 30 cycles of 96°C for 30 seconds, 50°C for 15 seconds and 60°C for 4 minutes. The reaction was then cooled to 4°C prior to purification.

Purification was performed by adding 80µl 75% isopropanol to the completed sequencing reaction. This was then mixed and left at room temperature for 30 minutes. The reaction was then centrifuged at 14,000 rpm for 20 minutes at room temperature. The supernatant was then removed and 250µl 75% isopropanol was added to the precipitate. The sample was mixed and centrifuged for 5 minutes at 14,000 rpm at room temperature. The supernatant was removed and the pellet dried at 75°C for 2 minutes.

Samples were then analysed on an ABI Prism 377 sequencer.

### Example 5 - Analysis of the growth hormone locus control region

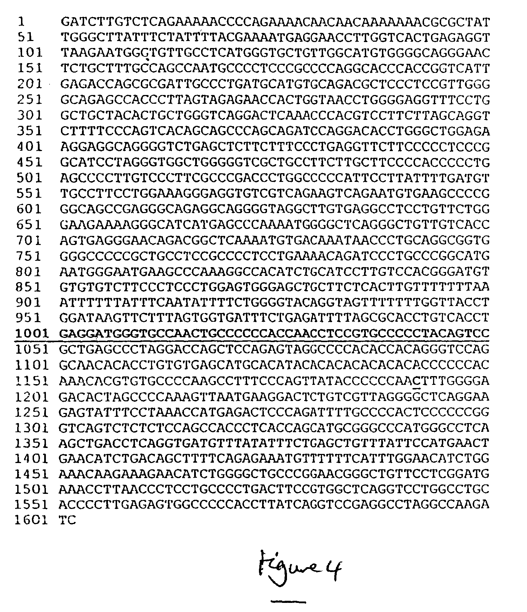

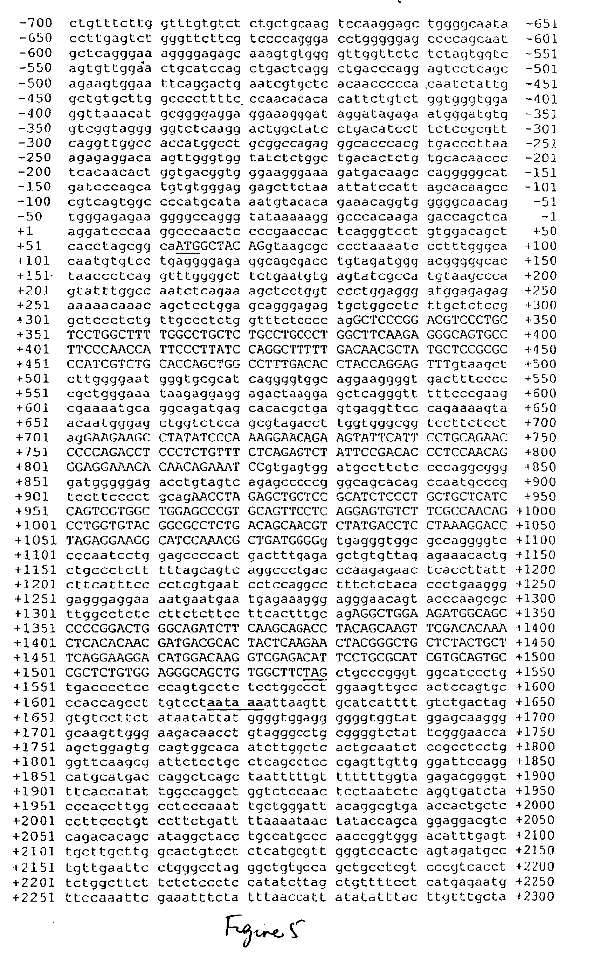

A DNA region approximately 14.5kb upstream of the human *GH1* gene is known to be involved in the tissue-specific and developmental control of *GH1* gene transcription [Jin *et al. Mol. Endocrinol.* 13: 1249-1266 (1999)]. This is known as the Locus Control Region (LCR) and its DNA sequence was obtained from GenBank (Accession Number: AF010280). Nucleotide numbering is based on the GH LCR reference sequence (Figure 4). The polymorphic site at position 1192 is marked in bold type and underlined. Part of this region was analysed by PCR and DHPLC.

Two overlapping PCR fragments spanning approximately 400bp were generated through the use of novel oligonucleotide primers designed by reference to the available DNA sequence: Fragment 1 primers were LCR15 (5' GTGCCCCAAGCCTTTCCC 3': 1159-1177) and
LCR13 (5' TGTCAGATGTTCAGTTCATGG 3': 1391-1412); and
fragment 2 primers were LCR25 (5' CCTCAAGCTGACCTCAGG 3': 1346-1363) and LCR23 (5' GATCTTGGCCTAGGCCTCG 3': 1584-1602).

PCR was performed using *Taq* Gold polymerase: 1µl patient genomic DNA was placed into a thin walled 0.2ml PCR tube or into one well of a 96-well micotitre plate. To this was added, 5µl 10x reaction buffer, 500ng of the appropriate primer pair (e.g. GH1DF and GH1DR), dATP, dTTP, dCTP and dGTP to a final concentration of 200µM, sterile water to a volume of 49.8µl followed by 0.2µl *Taq* Gold polymerase. The tube or microtitre plate was then placed in a Primus 96 thermal cycler (MWG Biotech) and cycled as follows: 12 minutes 95°C followed by 32 cycles of 95°C for 30 seconds, 58°C for 30 seconds and 72°C for 2 minutes. This was followed by a further incubation at 72°C for 10 minutes and the reaction was then cooled to 4°C prior to further analysis.

A one-tenth volume (5µl) was analysed on a 1.5% agarose gel to determine that the reaction had worked before denaturing high-pressure liquid chromatography (DHPLC) was performed. Analysis by DHPLC was performed as described in Example 3 with a melt temperature of 61°C.

### Example 6 - GH1 Gene Mutations and Polymorphisms

As can be seen from Table 7B, the selection characteristics according to the present invention have led to the characterisation and identification of some 34 different and novel variants in the *GH1* gene. In Table 7B, nucleotide numbering is based on the GH1 reference sequence shown in Figure 5, in which the five exons of the human *GH1* coding sequence are shown in upper case; the translation initiation (ATG) and termination codons (TAG) are underlined; the polyadenylation signal is shown in bold and is underlined; the 3' UTR boundary is at position +1642; and +1 = transcriptional initiation site. All numbering of mutational lesions, polymorphisms and oligonucleotide primers referred to in the text (with the exception of the Locus Control Region; see Figure 4) can be related to the *GH1* reference sequence.

The *GH1* reference sequence is derived from Chen *et al.* (1989) which was accessed through Genbank (Accession Number: J03071). Of 68 patients so far analysed, mutations have been found in 31 of them. All mutations detected were found in the heterozygous state with the exception of patient 37 (homozygous), patients 44, 55 and 57 (compound heterozygous for non-identical lesions *in trans*) (ie on different alleles) and patients 7, 32, 36, 48 and 70 who possess 2 or more mutations *in cis* (*ie* on the same allele).

Various novel promoter variants (nine single base-pair substitutions, two micro-deletions and an extensive gene conversion event) have been detected plus a common yet novel single base-pair substitution in the obligate AG dinucleotide of an acceptor splice site. In addition, a common novel variant within the LCR has been found. Further, a total of 21 novel single base-pair substitutions have been noted within the coding region of the *GH1* gene that change the amino acid encoded. Molecular modelling studies have suggested that they are located in regions of the GH molecule which either interact with the GH receptor or which may influence GH-GH receptor interactions.

One missense mutation (Lys41Arg) has been found in three unrelated patients on different haplotype backgrounds in two of these patients. This is consistent with recurrent mutation (i.e. independent mutation events) at this site. The IVS2 putative splicing mutation was found in a total of eight alleles in 7 apparently unrelated patients; since two distinct haplotypes are evident, at least two examples of this lesion are likely to be recurrent whilst the remainder may be identical-by-descent. Three examples of the promoter gene conversion event were also noted in this patient sample. These findings are very encouraging in terms of the prospect for the rapid detection of frequent pathological lesions.

During the course of our study, some 31 different putative polymorphisms were identified within the introns of the *GH1* gene. Most occurred only once and may be rare variants. All except the IVS4 T → A 1169 polymorphism, reported by Hasegawa *et al* (*ibid*), are novel. In Table 7A, the number is brackets is the number of times the rarer (second of the two) allelic variant was found in our study of 69 patients. IVS1-4 denote intron locations:

**Table 7A:**

| **Intronic polymorphisms in the human *GH1* gene** |
|---|
| IVS1 A/G 124 |
| IVS1 A/T 128 |
| IVS1 A/G 134 |
| IVS1 G/C 135 |
| IVS1 A/G 141 |
| IVS1 T/C 179 |
| IVS1 C/T 188 |
| IVS1 G/A 218 |
| Ins GAAA 251 |
| IVS1 T/C 281 (x 5) |
| IVS1 G/C 286 |
| IVS1 T/C 303 |
| |
| IVS2 A/G 565 |
| IVS2 A/G 580 |
| IVS2 A/G 622 |
| IVS2 T/C 665 |
| IVS2 G/A 620 |
| IVS2 A/G 670 |
| IVS2 G/A 676 |
| |
| IVS3 T/C 901 |
| |
| IVS4 C/T 1101 (x 5) |
| IVS4 T/A 1169 (x 45) |
| IVS4 C/T 1182 |
| IVS4 A/G 1189 |
| IVS4 T/C 1196 |
| IVS4 T/C 1208 |
| IVS4 C/T 1212 |
| IVS4 T/G 1216 (x 2) |
| IVS4 A/G 1219 |
| IVS4 A/G 1240 |
| IVS4 T/G 1302 |

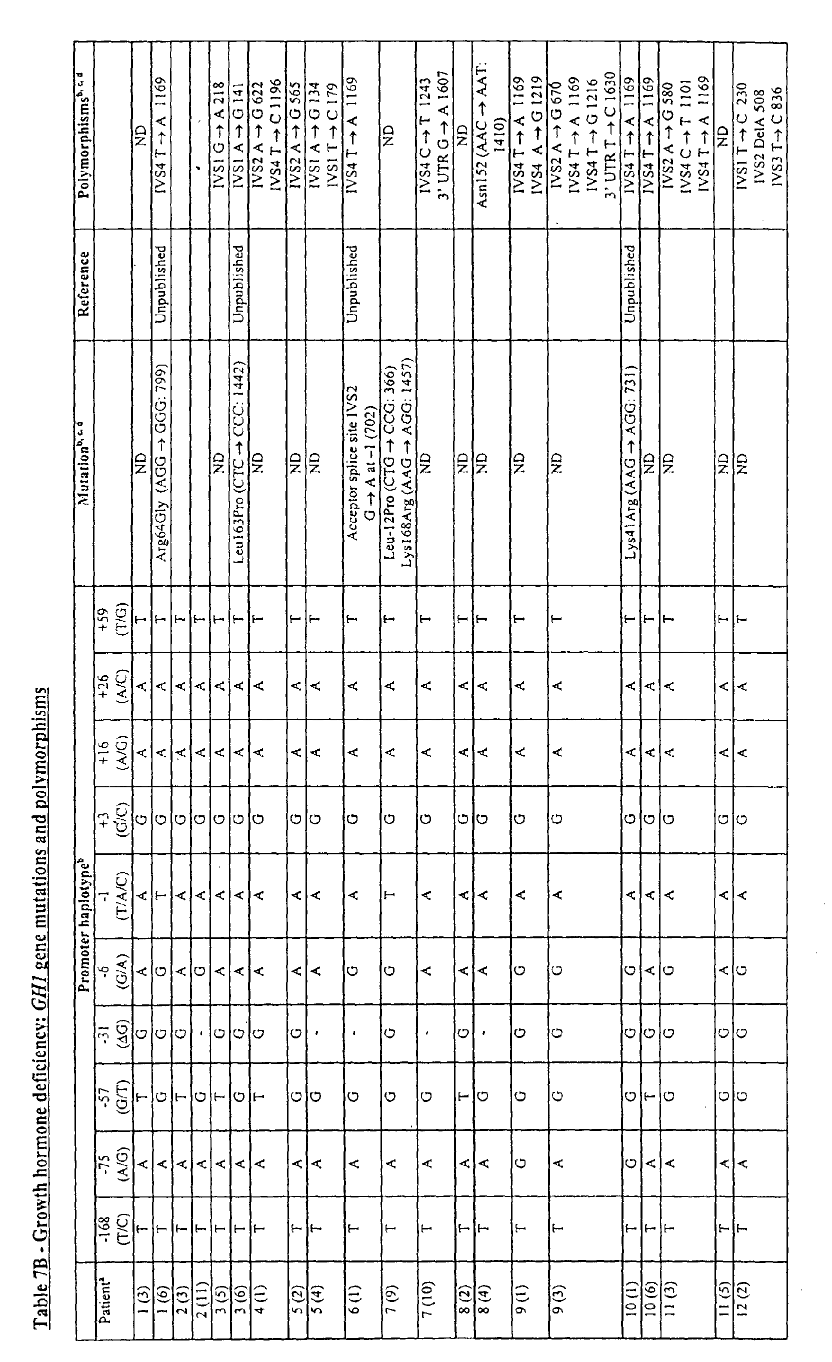

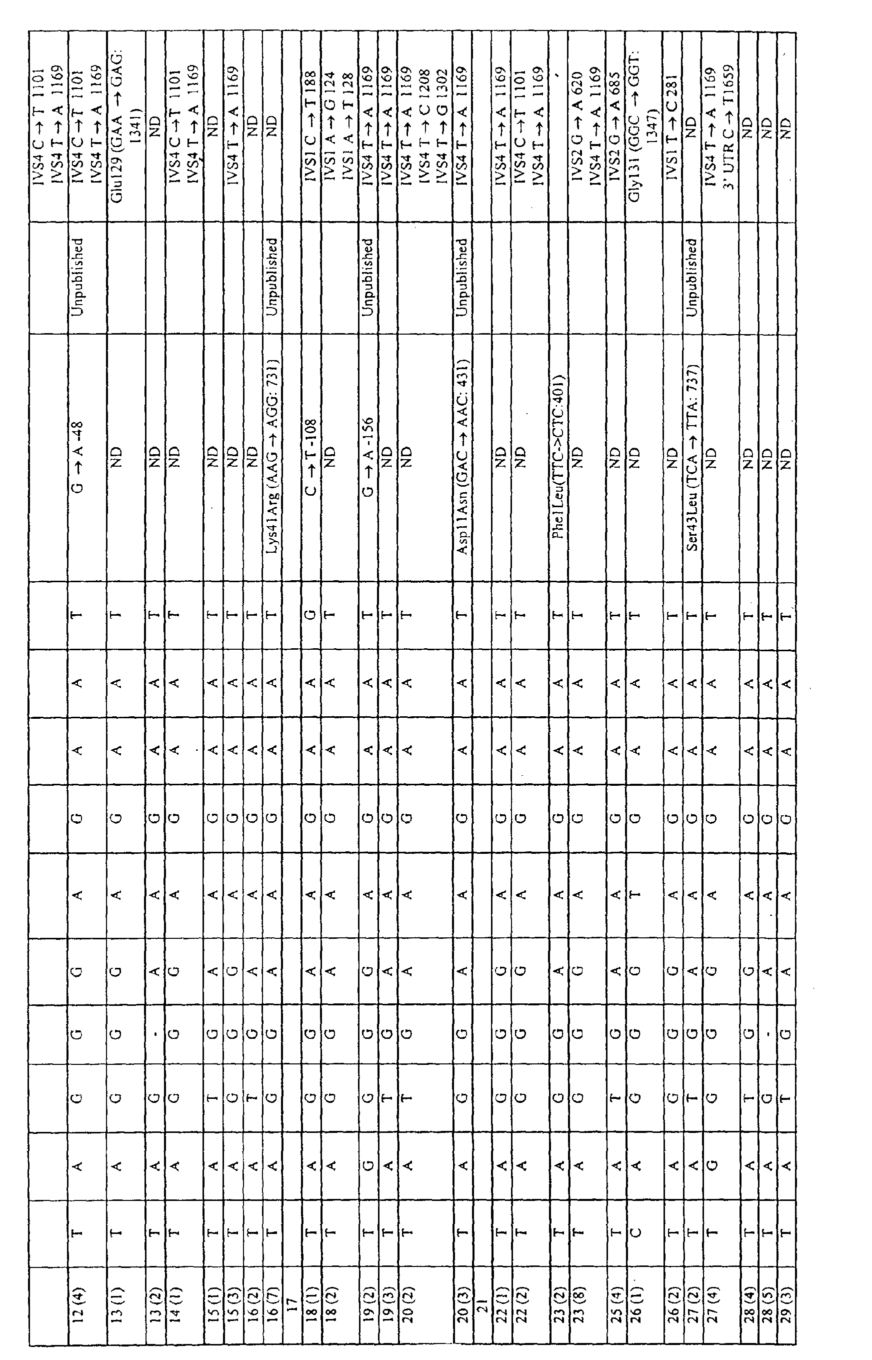

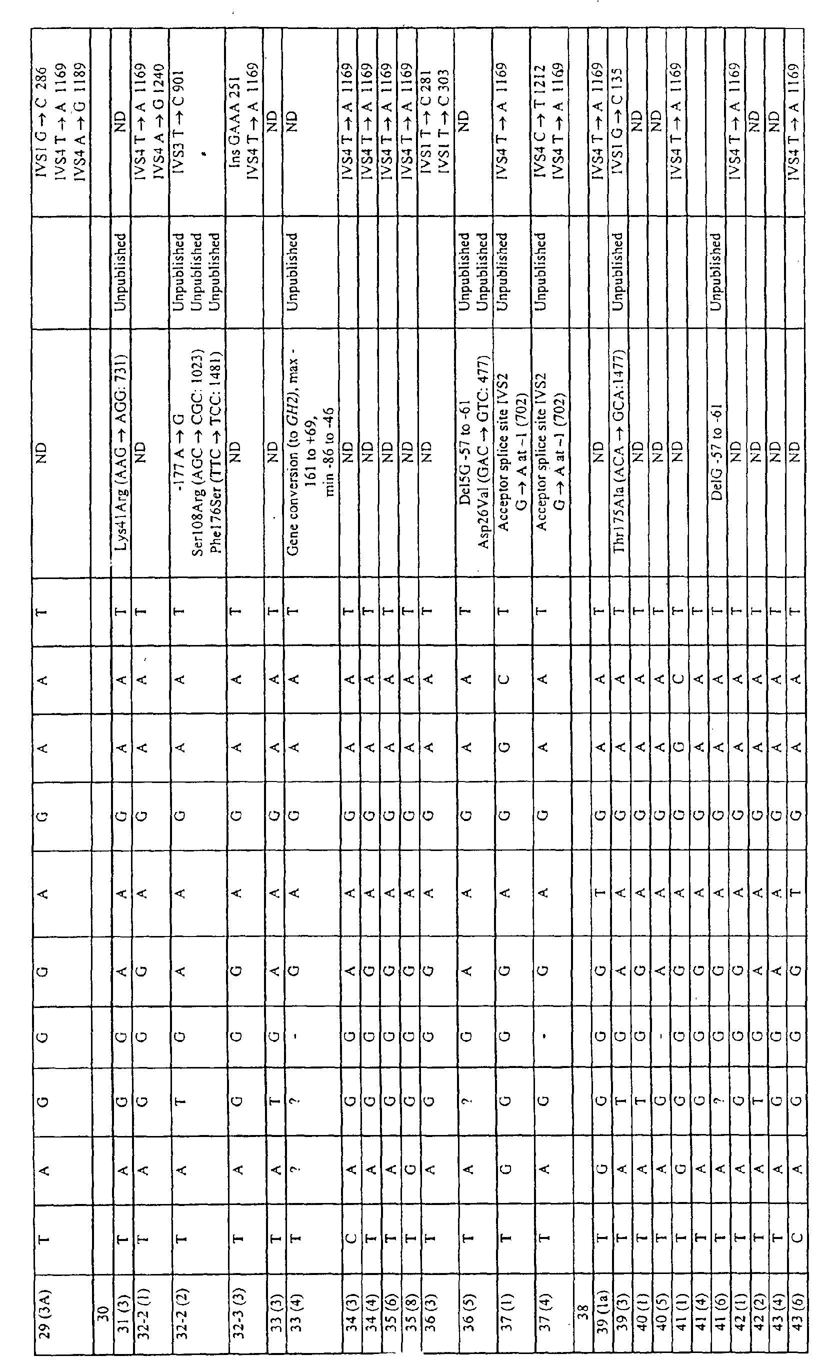

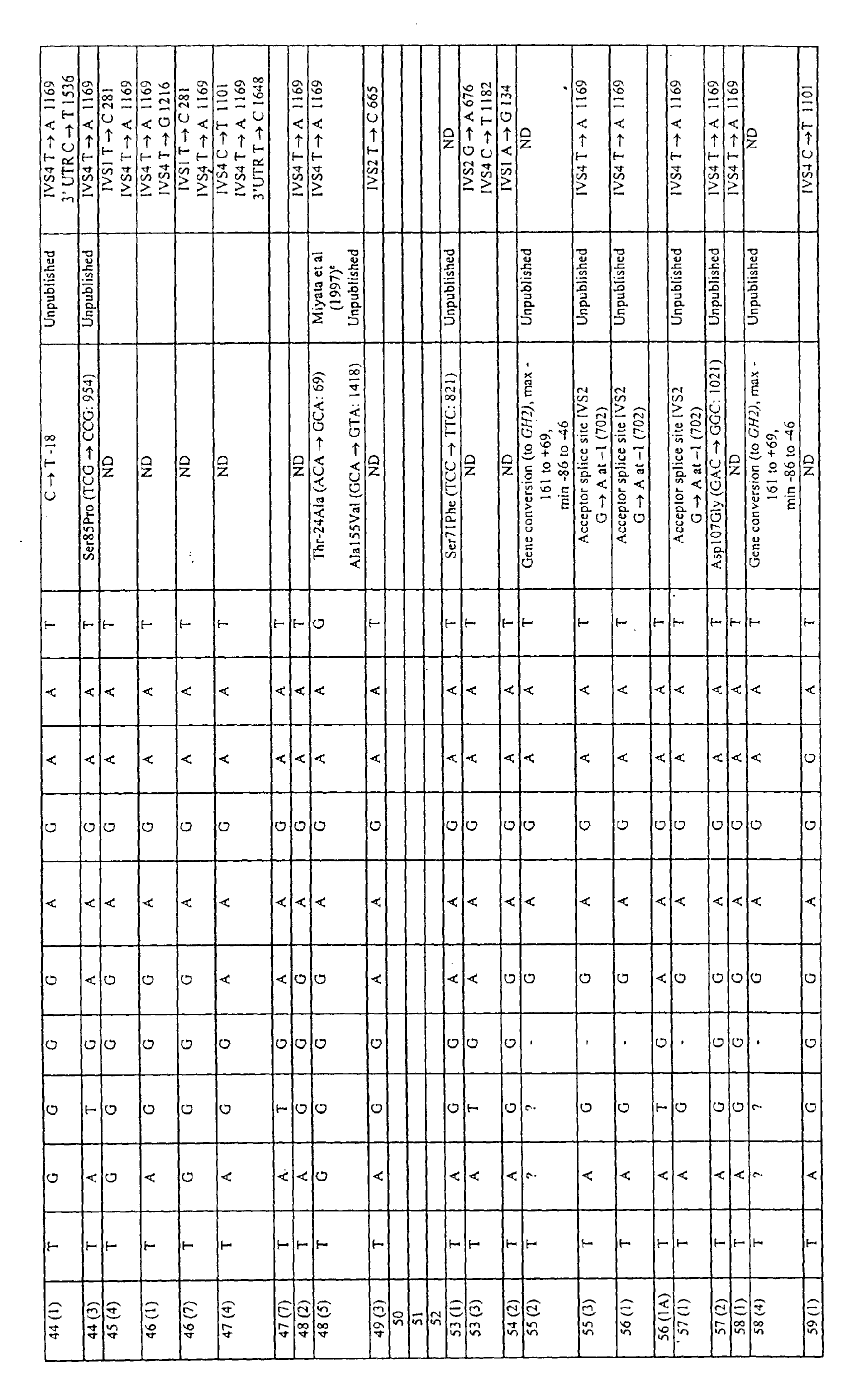

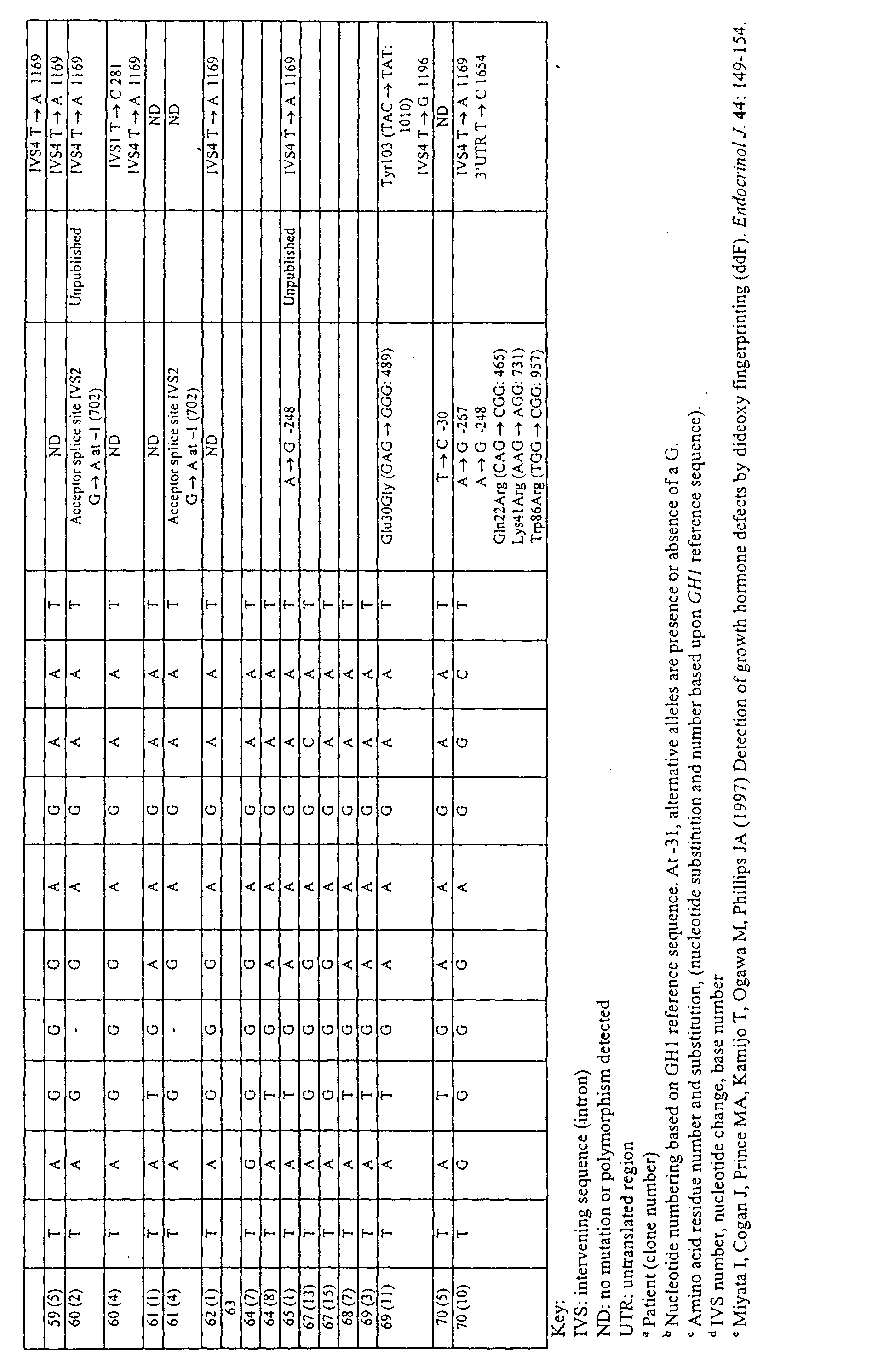

## Claims

1. A detection method for detecting a variation in *GH1* effective to act as an indicator of GH dysfunction in a patient, which detection method comprises the steps of:
(a) obtaining a test sample comprising a nucleotide sequence of the human *GH1* gene from the patient; and
(b) comparing the sequence obtained from the test sample with a standard sequence known to be that of the human *GH1* gene, wherein a difference between the test sample sequence and the standard sequence indicates the presence of a variation effective to act as an indicator of GH dysfunction (hereinafter "GH variant");
wherein the test sample is obtained from a patient exhibiting the following cumulative criteria:
(i) height velocity below the 25^{th} centile for age;
(ii) bone age delay according to the Tanner-Whitehouse scale of at least two years when compared with chronological age; and
(iii) no other disorder known to cause inclusion in criteria (i) to (ii) above.

2. A method according to claim 1,
wherein the test sample is obtained from a patient exhibiting the following cumulative criteria:
(i) height velocity below the 25^{th} centile for age;
(ii) bone age delay according to the Tanner-Whitehouse scale of at least two years when compared with chronological age;
(iii) no other disorder known to cause inclusion in criteria (i) to (ii) above; and
(iv) growth failure, defined as a growth pattern [delineated by a series of height measurements; Brook CDG, Ed., *Clinical Paediatric Endocrinology,* 3rd Ed., (1995) Blackwell Science. Chapter 9, p141] which, when plotted on a standard height chart [Tanner *et al*. *Arch. Dis. Child* 45: 755-762 (1970)], predicts an adult height for the patient which is outside the patient's estimated target adult height range, the estimate being based upon the heights of the patient's parents.

3. A method according to claim 1 or claim 2, wherein the detection method comprises PCR amplification of the *GH1* gene of the patient.

4. A method according to claim 3, wherein the detection method comprises PCR amplification of the entire *GH1* gene of the patient and nested PCR of overlapping constituent fragments of the *GH1* gene of the patient.

5. A method according to claim 3 or claim 4, wherein the detection method comprises PCR amplification of all or a fragment of genomic DNA spanning the Locus Control Region.

6. A method according to any preceding claim, wherein the detection method comprises mutational screening of the patient's *GH1* gene fragments by DHPLC.

7. A detection method for detecting a variation in *GH1* effective to act as an indicator of GH dysfunction in a patient, which detection method comprises the steps of:
(a) obtaining a test sample comprising a nucleotide sequence of the human *GH1* gene from the patient; and
(b) comparing the sequence obtained from the test sample with a standard sequence known to be that of the human *GH1* gene, wherein a difference between the test sample sequence and the standard sequence indicates the presence of a variation effective to act as an indicator of GH dysfunction (hereinafter "GH variant");
which detection method further comprises the use of one or more primer(s) selected from:

CTC CGC GTT CAG GTT GGC (GHD1F);
AGG TGA GCT GTC CAC AGG (GHD1R);
CTT CCA GGG ACC AGG AGC (GHD2R);
CAT GTA AGC CAA GTA TTT GGC C (GHD3F);
GGA GAA GGC ATC CAC TCA CGG (GHD4R);
TCA GAG TCT ATT CCG ACA CCC (GHD5F);
CGT AGT TCT TGA GTA GTG CGT CAT CG (GHD6R); and
TTC AAG CAG ACC TAC AGC AAG TTC G (GHD7F).

8. A method according to claim 7, wherein the test sample is obtained from a patient exhibiting the following cumulative criteria:
(i) height velocity below the 25^{th} centile for age;
(ii) bone age delay according to the Tanner-Whitehouse scale of at least two years when compared with chronological age; and
(iii) no other disorder known to cause inclusion in criteria (i) to (ii) above.

9. A method according to claim 7 or 8, wherein the detection method comprises PCR amplification of the *GH1* gene of the patient.

10. A method according to claim 9, wherein the detection method comprises PCR amplification of the entire *GH1* gene of the patient and nested PCR of overlapping constituent fragments of the *GH1* gene of the patient.

11. A method according to claim 7 ,8 or 9, wherein the detection method comprises PCR amplification of all or a fragment of genomic DNA spanning the Locus Control Region.

12. A GH variant detected by or detectable by a method according to any preceding claim.

13. A variant of *GH1*, which differs from *GH1* and is detectable by a method according to any preceding claim .

14. A variant of *GH1*, which differs from *GH1* and is detectable by a method according to any preceding claim, which variant is not detectable by methods wherein the patient selection criteria are based primarily on height and do not include the cumulative criteria (i) to (iii) as defined in claims 1, 2 and 8.

15. A GH variant or a variant of *GH1* according to any of claims 12to 14, which variant is selected from those characterised as unpublished in Example 6 and Table 7 herein.

16. A screening method for screening a patient suspected of having dysfunctional GH or dysfunctional GH axis, which screening method comprises the steps of:
(a) obtaining a test sample comprising a nucleotide sequence of the human *GH1* gene from the patient; and
(b) comparing a region of the sequence obtained from the test sample with the corresponding region of a predetermined sequence
wherein the predetermined sequence is selected from a *GH1* variant detectable by a method according to any of claims 1 to 11.

17. A screening method according to claim 16, wherein the predetermined sequence is an oligonucleotide having a nucleic acid sequence corresponding to a region of a variant *GH1* gene, which region incorporates at least one variation when compared with the corresponding region of the wild type sequence.

18. A screening method according to claim 16 or claim 17, wherein the variation is or includes any of those characterised as unpublished in Example 6 and Table 7 herein.

19. A screening method according to any of claims 16 to 18, wherein the test sample comprises genomic DNA.

20. A screening method for determining GH dysfunction, comprising:
(a) obtaining a first human tissue suspected of GH dysfunction; and
(b) comparing the *GH1* gene or *GH1* transcript, or fragment therefrom (*eg* cDNA), in the first human tissue to the corresponding gene, transcript or fragment of a *GH1* variant obtainable from a second human tissue derived from a human exhibiting the following cumulative criteria:
(i) height velocity below the 25^{th} centile for age;
(ii) bone age delay according to the Tanner-Whitehouse scale of at least two years when compared with chronological age; and
(iii) no other disorder known to cause inclusion in criteria (i) to (ii) above; and, optionally,
(iv) growth failure defined as a growth pattern [delineated by a series of height measurements; Brook CDG, Ed., *Clinical Paediatric Endocrinology,* 3rd Ed., (1995) Blackwell Science. Chapter 9, p141] which, when plotted on a standard height chart [Tanner *et al. Arch. Dis. Child* 45: 755-762 (1970)], predicts an adult height for the patient which is outside the patient's estimated target adult height range, the estimate being based upon the heights of the patient's parents.

21. A kit suitable for use in carrying out a screening method according to any of claims 16 to 20, which kit comprises:
(a) an oligonucleotide having a nucleic acid sequence corresponding to a region of a variant *GH1* gene, which region incorporates at least one variation from the corresponding wild-type sequence; and
(b) an oligonucleotide having a nucleic acid sequence corresponding to the wild-type sequence in the region specified in (a); and, optionally,
(c) one or more reagents suitable for carrying out PCR for amplifying desired regions of the patient's DNA.

22. A kit according to claim 21, wherein kit component (a) comprises a plurality of said oligonucleotides immobilised on a solid support.

23. A composition comprising a GH variant or a variant of *GH1* according to any of claims 12 to 15, in association with a pharmaceutically acceptable carrier therefor.

24. An isolated, purified or recombinant nucleic acid sequence selected from:
(a) a sequence encoding a variant of *GH1* according to any of claims 13 to 15;
(b) a sequence substantially homologous to or that hybridises to sequence (a) under stringent conditions; or
(c) a sequence substantially homologous to or that hybridises under stringent conditions to the sequence (a) or (b) but for the degeneracy of the genetic code; or
(d) an oligonucleotide specific for any of the sequences (a), (b) or (c).

25. A vector comprising a nucleic acid sequence according to claim 24.

26. A host cell comprising a vector according to claim 25, such as a bacterial host cell.

27. A process for preparing a variant of *GH1* according to any of claims 13 to 15, which process comprises:
(i) culturing a host cell according to claim 26; and
(ii) recovering from the culture medium the variant of *GH1* thereby produced.

28. A protein or amino acid sequence encoded or expressed by a sequence, vector, cell or culture medium as defined in any of claims 24 to 27.
